# FASCICULE DE BREVET EUROPEEN

(11) **EP 3 407 861 B1**
(45) Date de publication et mention de la délivrance du brevet: **04.12.2019**
(21) Numéro de dépôt: 17701523.7
(22) Date de dépôt: 27.01.2017
(51) Int. Cl.: A61K 8/37, A61Q 19/08, A61K 8/9783, A61K 36/39, C07C 69/757

(54) **ESTERS DE GLYCOL DE L'ACIDE DICAFEOYLQUINIQUE ET LEURS UTILISATIONS**
GLYKOLESTER VON DICAFFEOYLCHINASÄURE UND DEREN VERWENDUNGEN
GLYCOL ESTERS OF DICAFFEOYLQUINIC ACID AND USES THEREOF

(30) Priorité: 29.01.2016 FR 1650745; 26.10.2016 FR 1660402
(43) Date de publication de la demande: 05.12.2018
(73) Titulaire: Plant Advanced Technologies Pat, 54500 Vandoeuvre-les-Nancy (FR)
(72) Inventeur: HANNEWALD, Paul, 54990 Xeuilley (FR); BOURGAUD, Frédéric, 54500 Vandoeuvre Les Nancy (FR); MIGNARD, Benoît, 31300 Toulouse (FR); BOEGLIN, Damien, 67540 Ostwald (FR)
(74) Mandataire: IPAZ
(86) Numéro de dépôt international: PCT/EP2017/051749
(87) Numéro de publication internationale: WO 2017/129734

(56) Documents cités:
- EP-A1- 1 260 212
- WO-A1-01/33942
- WO-A1-2006/014028
- JP-A- 2012 041 297
- JP-A- 2015 199 674
- KIM HYOUNG-JA ET AL: "Isolation and Antioxidative Activities of Caffeoylquinic Acid Derivatives and Flavonoid Glycosides from Leaves of Sweet Potato (Ipomoea batatas L.)", EUNG'YONG YAGMUL HAGHOEJI = JOURNAL OF APPLIED PHARMACOLOGY, HAN'GUG EUNG'YONG YAGMUL HAGHOE, KR, vol. 15, no. 1, 30 mars 2007 (2007-03-30), pages 46-51, XP053020275, ISSN: 1225-6110

## Description

### DOMAINE DE L'INVENTION

La présente invention concerne des esters de glycol d'acides dicafeoylquiniques, notamment des esters de dipropylène glycol et des esters de propane 1,3 diol d'acides dicaféoylquiniques, et leur utilisation en tant que principes actifs dans des compositions cosmétiques. La présente invention concerne notamment l'utilisation en tant que principe actif des esters de dipropylène glycol d'acides dicaféoylquiniques (esters de DPG de DCQ) et des esters de propane 1,3 diol d'acides dicaféoylquiniques dans une composition cosmétique destinée à atténuer ou retarder l'apparition des signes du vieillissement intrinsèque ou extrinsèque de la peau, et un procédé de soin cosmétique de la peau utilisant une telle composition cosmétique.

### ART ANTERIEUR

La peau est la première barrière du corps humain. Elle protège les organes des différences de température, d'humidité, et des agressions de l'environnement extérieur, comme les rayons UV ou les agents polluants. Elle joue également un rôle important dans l'homéostasie, par exemple pour réguler la température corporelle. Cependant, des stimulations chimiques et physiques excessives (exposition au soleil, à la lumière ou aux UV ; stress et malnutrition) détériorent les fonctions normales de la peau et induisent son vieillissement. Ce vieillissement, dit « extrinsèque », entraîne des altérations cliniques telles que des rides profondes et la formation d'une peau ayant perdu sa fermeté, sa souplesse et son élasticité. Ces transformations sont dues essentiellement à des changements histopathologiques, tels qu'une excessive modification du tissu élastique dans le derme supérieur et une dégénérescence quantitative et qualitative des fibres de collagène.

De façon concomitante, le vieillissement dit « intrinsèque », « physiologique », « normal » ou « chronobiologique », est la conséquence d'une sénescence programmée où interviennent des facteurs endogènes. Ce vieillissement intrinsèque provoque notamment un ralentissement du renouvellement des cellules de la peau, les kératinocytes, ce qui se traduit essentiellement par l'apparition d'altérations cliniques telles que la réduction du tissu adipeux sous-cutané et l'apparition de fines rides ou ridules, et par des changements histopathologiques tels qu'une augmentation du nombre et de l'épaisseur des fibres élastiques, une perte de fibres verticales de la membrane du tissu élastique, et la présence de grands fibroblastes irréguliers dans les cellules de ce tissu élastique. La présente invention s'intéresse à ces deux types de vieillissement, aussi bien intrinsèque qu'extrinsèque.

La recherche de nouvelles molécules ou ingrédients actifs utilisables en cosmétique est une nécessité pour pouvoir développer des produits efficaces pour donner un aspect plus jeune à la peau, atténuer les rides, lisser la peau et rendre au teint son éclat. Les laboratoires rivalisent donc d'innovation à la recherche d'actifs de plus en plus élaborés.
Plusieurs catégories d'ingrédients actifs anti-vieillissement sont aujourd'hui commercialisées :
- les agents anti-oxydants (vitamines A, C, E, oligo-éléments, végétaux, algues) luttent contre les radicaux libres, améliorent le relief de la peau et réparent les dommages causés par la pollution. Plus particulièrement, le rétinol, forme active de la vitamine A, lisse les ridules peu profondes et augmente l'épaisseur de la peau en stimulant le renouvellement cellulaire,
- les alpha hydroxyacides (AHA), présents dans les agrumes, le raisin et la canne à sucre, bénéficient d'un pouvoir exfoliant qui élimine les cellules mortes de l'épiderme, hydrate et lisse les traits pour raviver l'éclat du teint,
- de nouvelles formules permettent également de stimuler la production de collagène et d'élastine, de favoriser la régénération des tissus façon « laser » et des soins « botox-like » inspirés de la toxine botulique pour atténuer la contraction des traits.

La demande de brevet US 2004/0170581 décrit un extrait de chicorée comprenant des DCQ ainsi que l'utilisation d'un tel extrait pour son activité anti-âge. Il est précisé que l'extrait de chicorée combiné à de l'acide rétinoïque stimule l'activité enzymatique de la glucose-6-phosphate déshydrogénase qui est impliquée dans la lutte contre les radicaux libres et donc contre le vieillissement. Ainsi, selon le document US 2004/0170581, un extrait de chicorée comprenant des DCQ a un fort potentiel dans la lutte contre le stress oxydatif et le vieillissement, et est impliqué dans le renouvellement des constituants vitaux de la peau, tels que le collagène, l'élastine et les glycoprotéines.

Le vieillissement de la peau entraîne de nombreux changements histopathologiques, modifiant sa beauté et la qualité visible de celle-ci ; il peut induire en effet une diminution de l'hydratation, une modification de la différentiation des kératinocytes de l'épiderme, et un ralentissement de leur prolifération. Les ingrédients actifs et mécanismes décrits ci-dessus permettent dans une certaine mesure de réparer, a posteriori, les altérations de la peau liées à son vieillissement. Cependant, il existe un besoin de prévenir le vieillissement, notamment en retardant les signes du vieillissement intrinsèque ou extrinsèque de la peau.

Le stress oxydant (sous la forme de radiations UV, de pollution ou de radicaux libres) est à présent reconnu comme l'un des principaux déterminants impliqués dans les phénomènes du vieillissement (F. J. Kelly. Occup. Environ. Med., 2003, 60: 612-616). Le vieillissement serait ainsi relié à une accumulation progressive et irréversible de dommages causés par le stress oxydant de par la diminution avec l'âge de l'activité des systèmes de défenses (Kevin C. Kregel, Hannah J. Zhang. Am J Physiol Regul Integr Comp Physiol, 2007, 292:R18-R36). Les actifs permettant de renforcer l'un ou l'autre de ces mécanismes de défense ont donc des effets préventifs anti-âges potentiels très intéressants.

Un agent hormétique, ou hormétine, est une molécule ou un traitement générant un stress de faible intensité, sans dommages cellulaires importants, mais qui induit une réponse protectrice et antioxydante afin d'assurer l'homéostasie de la cellule. L'effet hormétique, ou hormesis, est donc une stimulation positive des mécanismes de défense de l'organisme par l'application de stress légers à modérés. Cette stimulation améliore ainsi les capacités fonctionnelles des mécanismes d'adaptation, de maintenance et de réparation, et peut ainsi avoir une grande variété d'effets anti-âge *(*Suresh I. S. Rattan. The Journals of Gerontology Series A: Biological Sciences and Medical Sciences, 2004, 59:B705-B709). L'effet hormétique peut être représenté comme un entrainement et une préparation des cellules au stress oxydant. L'organisme est alors mieux à même de répondre en limitant au maximum les dommages encourus lors de l'exposition future à ces stress. L'utilisation d'agents hormétiques, ou hormétines, a ainsi un effet avéré dans la prévention et donc le retardement du vieillissement par l'activation des mécanismes de défense cellulaire (Zsolt Radak, Hae Young Chung, Sataro Goto. Biogerontology, 2005, 6: 71-75).

Pour être qualifiée d'hormétine, une substance doit induire une réponse cellulaire défensive notamment par la modification de l'expression de certains gènes, appelés vitagènes (S. I. Rattan. Ann. N. Y. Acad. Sci. 1998, 854: 54-60*),* dans lesquels on retrouve notamment les gènes codants pour l'Hème-Oxygénase 1 (D. Gems, L. Partridge. Cell Metabolism, 2008, 7: 200-204*).*
Ainsi, la lutte contre le vieillissement et l'effet anti-âge dépendent de l'efficacité des systèmes de maintenance et de réparation cellulaire. Parmi ces systèmes se trouvent notamment les protéines de défense au stress oxydant telles que l'Hème-oxygénase 1 (HO-1) ou la protéine de choc thermique 70 (HSP70).

La protéine HO-1 (codée par le gène HMOX) est impliquée dans la dégradation de l'hème et est connue pour démontrer un rôle protecteur contre le stress oxydant (*Morse and Choi, 2005 ; Vile et al.,* 1994). Le gène est induit rapidement après l'exposition aux radicaux libres, et son rôle protecteur est essentiellement dû à sa capacité à dégrader l'hème (ayant des propriétés pro-oxydante) en monoxyde de carbone et biliverdine, précurseur d'un puissant métabolite antioxydant, la bilirubine.

Par ailleurs, HMOX (gène codant pour HO-1) est un gène cible du facteur de transcription Nrf2. La réponse antioxydante du facteur de transcription Nrf2 est donc considérée comme «la défense cellulaire primaire contre les effets cytotoxiques du stress oxydatif ». L'activation de Nrf2 résulte dans l'induction de nombreuses protéines cytoprotectrices, à savoir HO-1, mais aussi HSP70, NAD(P)H, la glutamate cystéine ligase, la glutathion S transférase pi, l'UDP-glucuronosyltransférase 1A6 et l'inhibition de l'oxyde nitrique synthase. Toutes les protéines induites sont des protéines ayant des actions cytoprotectrices. Ainsi, la protéine HSP70 (codée par le gène HSPA1A) est une protéine de choc thermique induite en cas de stress et exerçant une action protectrice sur d'autres protéines (*Murphy, 2013*)*.* Elle contrôle également des médiateurs essentiels de la machinerie apoptotique. La NAD(P)H déshydrogénase, quinone 1 encodée par le gène NQO1 exerce quant à elle, une action détoxifiante en permettant la formation d'hydroquinones à partir de quinones, empêchant la formation d'espèces radicalaires. Elle est également induite en réponse à un stress cellulaire (UV, métaux lourds, agents pro-oxydants, etc.) (*Nguyen et al., 2009*)*.* La glutamate cystéine ligase (codée par le gène GCLM), et la glutathion S transférase pi (codée par le gène GSTP1) sont impliquées respectivement dans la synthèse du glutathion et dans sa conjugaison à des substances endogènes ou exogènes en vue de rétablir le statut rédox de la cellule (*Lu et al., 2009 ; Strange et al., 2001*)*.* L'UDP-glucuronosyltransférase 1A6 (codée par le gène UGT1A6) induit la conjugaison de l'acide glucuronique à d'autres substances endo- ou xénobiotiques afin de faciliter leur excrétion (*Buckley* & *Klaassen, 2009*)*.* Enfin, l'oxyde nitrique synthase (codée par le gène NOS2) est impliquée dans la production d'espèces réactives de l'azote (*reactive nitrogen species* ou RNS) pouvant être produites lors d'un stress oxydatif. L'activation de la voie dépendante de Nrf-2 diminue l'induction du gène NOS2 consécutive à un stress (*Petri et al., 2012*) et donc la production de ces espèces réactives de l'azote. L'induction de Nrf-2, ayant pour conséquence l'induction des gènes HMOX, HSPA1A, NQO1, GCLM, GSTP1, et UGT1A6, et l'inhibition du gène NOS2, contribue donc à la réponse hormétique de la cellule.

A titre d'exemple, les hormétines moléculaires les mieux documentées sont le curcumin et l'acide férulique, dont l'application sur fibroblastes humain a pour conséquence de générer une surproduction de radicaux libres, d'induire certaines enzymes protectrices ou anti-oxydantes comme HSP70 ou HO-1, et de déclencher une réponse anti-oxydante globale via la voie de signalisation dépendante de Nrf-2 (Lima et al., Mol. Nutr. Food Res., 54, 1-13, 2010*;* Calabrese et al., Clinics in Dermatology, 26, 358-363, 2008)*.*

Cependant, il existe encore un besoin pour de nouvelles substances hormétiques, capables d'avoir un rôle de prévention du vieillissement, notamment cutané, qui peuvent être obtenues facilement, et qui répondent à l'actuel intérêt des consommateurs envers les ingrédients d'origine végétale.

### RESUME DE L'INVENTION

Dans le cadre de la présente invention, il a été découvert qu'un extrait végétal comprenant des esters de glycol d'acides dicaféoylquiniques, notamment des esters de DPG de DCQ et des esters de propane 1,3 diol de DCQ, possède un effet hormétique sur les fibroblastes et peut donc être utilisé dans des applications cosmétiques anti-âge. Plus précisément, les inventeurs ont montré que les esters de DPG de DCQ présents dans cet extrait possèdent un effet hormétique sur les fibroblastes, contrairement aux DCQ eux-mêmes, qui n'ont pas cette activité. Les inventeurs ont également montré que des esters de glycol de DCQ, notamment des esters de DPG de DCQ et des esters de propane 1,3 diol de DCQ, présents dans un extrait végétal, modifient de façon significative l'expression des gènes impliqués dans un stress oxydant, la pigmentation cutanée et la jonction dermo-épidermique dans des cultures cellulaires de fibroblastes humains ou d'épidermes humains reconstitués mélanisés.

Dans un premier aspect, la présente invention concerne donc un composé ester de glycol d'acide dicaféoylquinique de formule générale (I) : 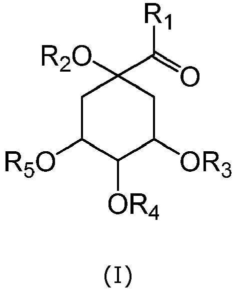 dans laquelle
- R₁ représente un radical choisi dans le groupe constitué par les radicaux de formule (IIa) à (IIe), (III), (IVa) à (IVb) et (Va) à (Vd) suivantes : 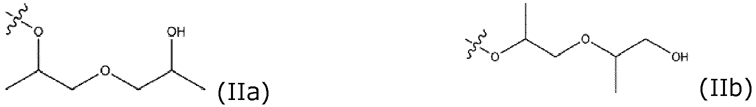 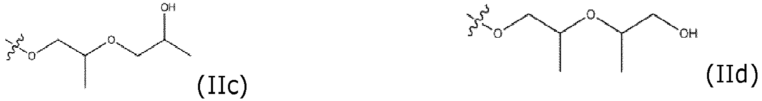 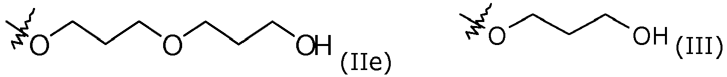 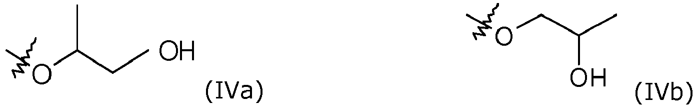 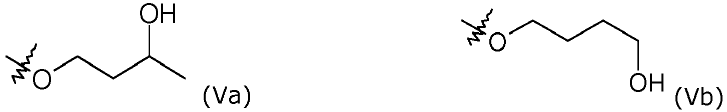 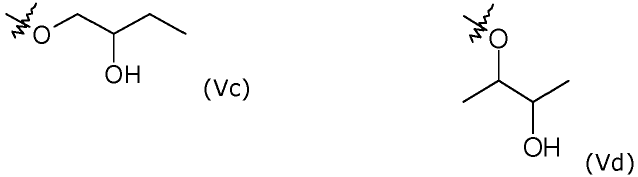 et
- deux quelconques des radicaux R₂, R₃, R₄ et R₅ représentent un groupement caféoyle, les deux autres représentant un atome d'hydrogène.

Dans un second aspect, l'invention concerne un extrait végétal comprenant au moins un composé selon l'invention.
Dans un troisième aspect, l'invention concerne un procédé de préparation dudit extrait végétal comprenant au moins un composé selon l'invention, comprenant les étapes suivantes:
a) Culture de plantes synthétisant des acides dicafeoylquiniques (DCQ),
b) Extraction solide/liquide des racines des plantes issues de l'étape a) dans du glycol ou dans une solution de glycol.

Dans un quatrième aspect, la présente invention concerne un extrait susceptible d'être obtenu par ledit procédé selon l'invention.
Dans un autre aspect, la présente invention concerne une composition cosmétique comprenant en tant qu'agent actif au moins un composé selon l'invention ou un extrait végétal selon l'invention, et avantageusement un excipient.
Dans encore un autre aspect, l'invention concerne l'utilisation comme agent cosmétique d'au moins un composé selon l'invention ou d'un extrait végétal selon l'invention ou d'une composition cosmétique selon l'invention.

La présente invention concerne également une méthode de soin cosmétique de la peau visant à prévenir ou retarder l'apparition des effets du vieillissement de la peau, caractérisée en ce qu'elle comprend l'application, sur au moins une partie de la peau du corps ou du visage, de la composition cosmétique selon l'invention.

### DESCRIPTION DES FIGURES

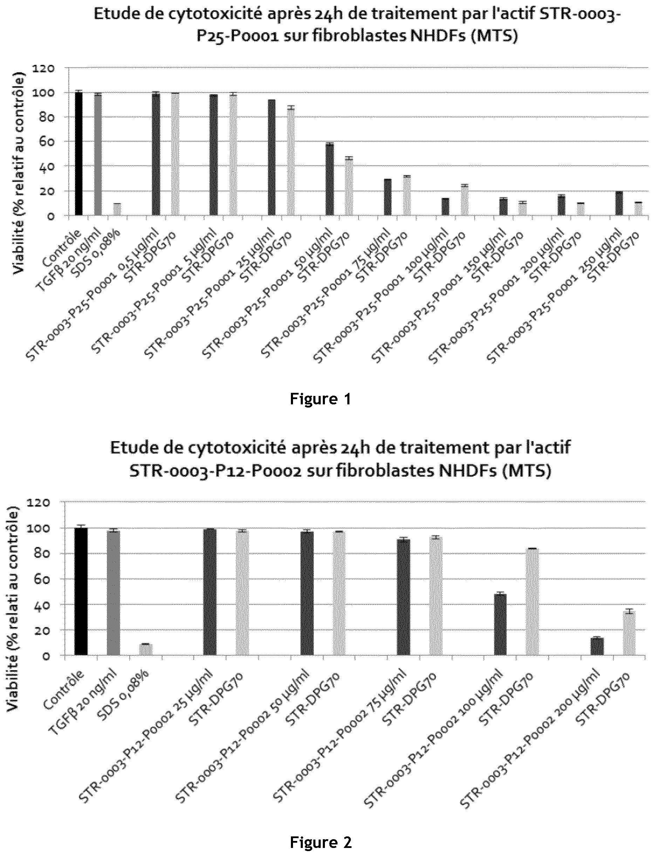

Figure 1. Mesures de la viabilité de fibroblastes NHDFs après 24h de traitement avec différentes concentrations d'un extrait *d'Ipomoea batatas* selon l'invention (STR-0003-P25-P0001, en noir), ou de son solvant seul à la même concentration (STR-DPG70, en gris), ou avec du TGF-β₁ (contrôle positif de la variation d'expression des gènes impliqués dans le vieillissement cellulaire ou cutané). Les pourcentages de viabilité ont été calculés par rapport au contrôle négatif non traité (Contrôle) fixé à 100% et au contrôle positif de cytotoxicité (SDS à 0,08%). Les histogrammes représentent la moyenne de 3 réplicats indépendants et la barre d'erreur correspond à l'écart-type autour de la moyenne.
Figure 2. Mesures de la viabilité de fibroblastes NHDFs après 24h de traitement avec différentes concentrations d'un extrait *d'Ipomoea batatas* selon l'invention (STR-0003-P12-P0002, en noir) ou de son solvant seul à la même concentration (STR-DPG70, en gris), ou avec du TGF-β₁ (contrôle positif de la variation d'expression des gènes impliqués dans le vieillissement cellulaire ou cutané). Les pourcentages de viabilité ont été calculés par rapport au contrôle non traité (CTL) fixé à 100%, et au contrôle positif de cytotoxicité (SDS à 0,08%). Les histogrammes représentent la moyenne de 3 réplicats indépendants et la barre d'erreur correspond à l'écart-type autour de la moyenne.

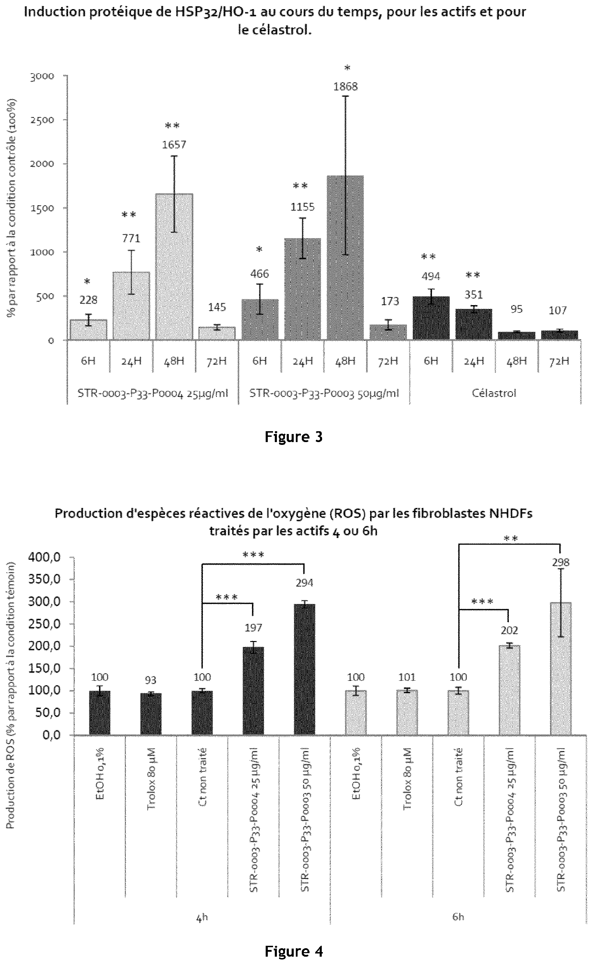

Figure 3. Quantification de la protéine HO-1/HSP₃₂ dans des fibroblastes traités avec les extraits selon l'invention STR-0003-P25-P0003 et STR-0003-P12-P0004 durant 6h, 24h, 48h, et 72h. Le contrôle positif de l'expérience induisant l'expression de la protéine HO-1 est le célastrol utilisé à 250 nM. Le graphique représente la moyenne des mesures faites sur les lysats issus de 3 cultures indépendantes par condition, ainsi que l'écart-type. L'analyse statistique menée est un test t de Student permettant de comparer l'effet individuel de chacun des traitements par rapport à la condition contrôle (contrôle non traité pour les deux actifs et contrôle DMSO 0,05% pour le célastrol) fixée à 100% (* : 0,01 < p < 0,05 ; ** : 0,001< p < 0,01). Les valeurs 0,01 < p < 0,05 ont été considérées comme significatives et les valeurs 0,001 < p < 0,01 comme hautement significatives.
Figure 4. Quantification de la production d'espèces réactives de l'oxygène (ROS) suite au traitement des fibroblastes NHDFs par deux extraits *d'Ipomoea batatas* selon l'invention STR-0003-P33-P0003 et STR-0003-P33-P0004 durant 4 ou 6 heures. Les données ont été normalisées par rapport à la viabilité des cellules évaluée par un test MTS après le dosage des ROS. Le graphique représente la moyenne des valeurs obtenues à partir de 3 cultures indépendantes par condition, ainsi que l'écart-type. L'analyse statistique menée est un test t de Student permettant de comparer l'effet individuel de chacun des traitements par rapport à la condition contrôle (contrôle non traité pour les deux actifs et EtOH 0.1% pour le Trolox) fixée à 100%. (* : 0,01 < p < 0,05 ; ** : 0,001< p < 0,01 ; *** : p < 0,001). Les valeurs p < 0,05 ont été considérées comme significatives, les valeurs 0,001 < p < 0,01 comme hautement significatives et les valeurs p < 0,001 comme très hautement significatives.

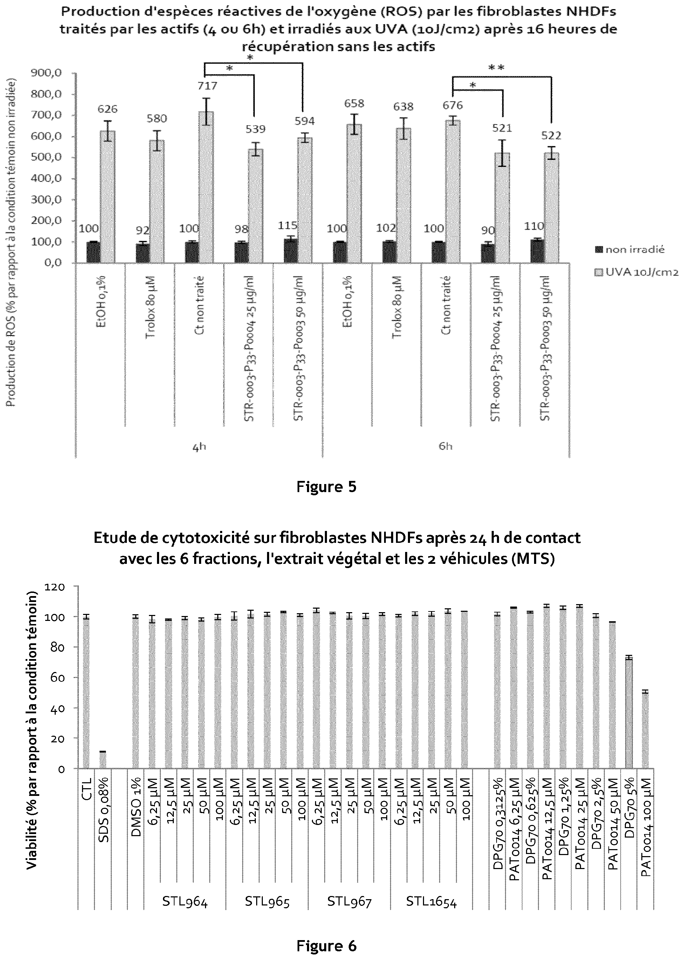

Figure 5. Quantification de la production d'espèces réactives de l'oxygène (ROS) par les fibroblastes NHDFs suite à un stress UVA (10 J/cm2). Les cellules ont été pré-traitées 4 ou 6 h par les extraits STR-0003-P33-P0003 et STR-0003-P33-P0004 et ensuite ont récupéré 16 heures dans un milieu frais. Au terme de ces 16 heures, le stress UVA a été réalisé et la production de ROS a été quantifiée par mesure de la fluorescence. Les données de fluorescence ont été normalisées par rapport à la viabilité des cellules évaluée par MTS après le stress UVA et le dosage des ROS. Le graphique représente la moyenne des valeurs obtenues à partir de 3 cultures indépendantes par condition, ainsi que l'écart-type. L'analyse statistique menée est un test t de Student permettant de comparer l'effet individuel de chacun des traitements par rapport à la condition contrôle (contrôle non traité pour les deux actifs et EtOH 0,1% pour le Trolox) fixée à 100%. (* : 0,01 < p < 0,05 ; ** : 0,001< p < 0,01). Les valeurs p < 0,05 ont été considérées comme significatives, les valeurs 0,001 < p < 0,01 comme hautement significatives et les valeurs p < 0,001 comme très hautement significatives.
Figure 6. Mesures de la viabilité de fibroblastes NHDFs après 24h de traitement avec différentes concentrations en fractions et véhicules. Les pourcentages de viabilité ont été calculés par rapport au contrôle non traité (CTL) fixé à 100%. Les fractions STL964 (comprenant majoritairement du 3,4-DCQ), STL 965 (comprenant majoritairement du 3,5-DCQ), STL 967 (comprenant majoritairement du 4,5-DCQ) et STL1654 (comprenant majoritairement des esters de DPG de DCQ), ont été préparées dans du DMSO (diméthylsulfoxyde). L'extrait PAT0014 correspond à un extrait *d'Ipomoea batatas* selon l'invention préparé dans du DPG70 (Dipropylène glycol à 70% dans de l'eau v/v).

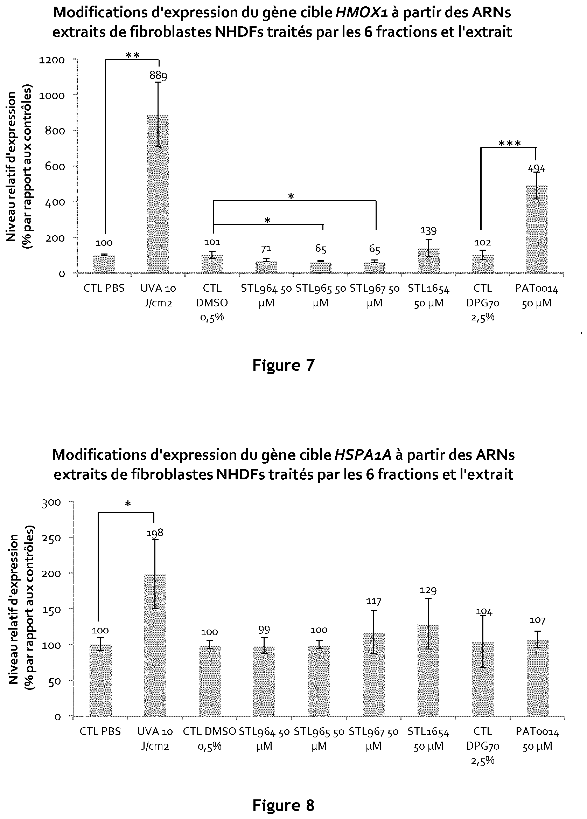

Figure 7. Niveau d'expression du gène HMOX1 dans des fibroblastes NHDFs traités pendant 24h par du PBS (contrôle « CTL PBS »), du DMSO (contrôle « CTL DMSO »), les fractions STL964 (comprenant majoritairement des isomères de DCQ), STL 965 (comprenant majoritairement du 3,5-DCQ), STL 967 (comprenant majoritairement des isomères de DCQ) et STL1654 (comprenant majoritairement des esters de DPG de DCQ), du DPG à 70% dans de l'eau v/v (contrôle « CTL DPG70 ») ou par l'extrait PAT0014 (correspondant à un extrait selon l'invention) ou dans des fibroblastes NHDFs irradiés aux UVA, après 6h de récupération. Les fractions STL 964, STL 965, STL 967 et STL 1654 étant préparées dans du DMSO, leur fraction de contrôle est « CTL DMSO ». De même, l'extrait PAT0014 est préparé dans du DPG à 70%, sa fraction de contrôle correspond donc à « CTL DPG70 ». Les résultats sont exprimés en pourcentage relatif par rapport aux contrôles respectifs. L'analyse statistique effectuée est basée sur un test t-student comparant individuellement chaque traitement à son contrôle (* : p<0,05 ; ** : 0,001<p<0,01 et *** : p<0,001). Les valeurs p < 0,05 ont été considérées comme significatives, les valeurs 0,001 < p < 0,01 comme hautement significatives et les valeurs p < 0,001 comme très hautement significatives.
Figure 8. Niveau d'expression du gène HSPA1A dans des fibroblastes NHDFs traités pendant 24h par du PBS (contrôle « CTL PBS »), du DMSO (contrôle « CTL DMSO »), les fractions STL964 (comprenant majoritairement du 3,4-DCQ), STL 965 (comprenant majoritairement du 3,5-DCQ), STL 967 (comprenant majoritairement du 4,5-DCQ) et STL1654 (comprenant majoritairement des esters de DPG de DCQ), du DPG à 70% dans de l'eau v/v (contrôle « CTL DPG70 ») ou par l'extrait PAT0014 (correspondant à un extrait selon l'invention) ou dans des fibroblastes NHDFs irradiés aux UVA, après 6h de récupération. Les fractions STL 964, STL 965, STL 967 et STL 1654 étant préparées dans du DMSO, leur fraction de contrôle est « CTL DMSO ». De même, l'extrait PAT0014 est préparé dans du DPG à 70%, sa fraction de contrôle correspond donc à « CTL DPG70 ». Les résultats sont exprimés en pourcentage relatif par rapport aux contrôles respectifs. L'analyse statistique effectuée est basée sur un test t-student comparant individuellement chaque traitement à son contrôle (* : p<0,05 ; ** : 0,001<p<0,01 et *** : p<0,001). Les valeurs p < 0,05 ont été considérées comme significatives, les valeurs 0,001 < p < 0,01 comme hautement significatives et les valeurs p < 0,001 comme très hautement significatives.

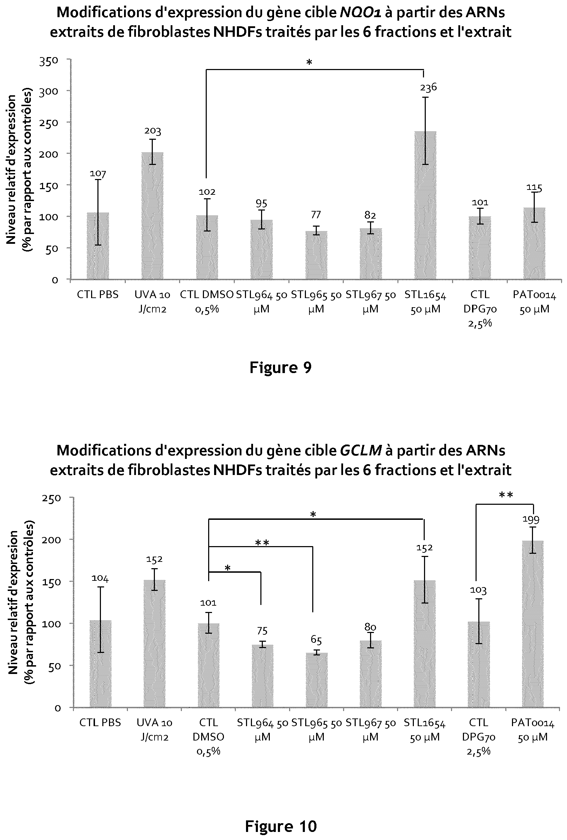

Figure 9. Niveau d'expression du gène NQO1 dans des fibroblastes NHDFs traités pendant 24h par du PBS (contrôle « CTL PBS »), du DMSO (contrôle « CTL DMSO »), les fractions STL964 (comprenant majoritairement du 3,4-DCQ), STL 965 (comprenant majoritairement du 3,5-DCQ), STL 967 (comprenant majoritairement du 4,5-DCQ) et STL1654 (comprenant majoritairement des esters de DPG de DCQ), du DPG à 70% dans de l'eau v/v (contrôle « CTL DPG70 ») ou par l'extrait PAT0014 (correspondant à un extrait selon l'invention) ou dans des fibroblastes NHDFs irradiés aux UVA, après 6h de récupération. Les fractions STL 964, STL 965, STL 967 et STL 1654 étant préparées dans du DMSO, leur fraction de contrôle est « CTL DMSO ». De même, l'extrait PAT0014 est préparé dans du DPG à 70%, sa fraction de contrôle correspond donc à « CTL DPG70 ». Les résultats sont exprimés en pourcentage relatif par rapport aux contrôles respectifs. L'analyse statistique effectuée est basée sur un test t-student comparant individuellement chaque traitement à son contrôle (* : p<0,05 ; ** : 0,001<p<0,01 et *** : p<0,001). Les valeurs p < 0,05 ont été considérées comme significatives, les valeurs 0,001 < p < 0,01 comme hautement significatives et les valeurs p < 0,001 comme très hautement significatives.
Figure 10. Niveau d'expression du gène GCLM dans des fibroblastes NHDFs traités pendant 24h par du PBS (contrôle « CTL PBS »), du DMSO (contrôle « CTL DMSO »), les fractions STL964 (comprenant majoritairement du 3,4-DCQ), STL 965 (comprenant majoritairement du 3,5-DCQ), STL 967 (comprenant majoritairement du 4,5-DCQ) et STL1654 (comprenant majoritairement des esters de DPG de DCQ), du DPG à 70% dans de l'eau v/v (contrôle « CTL DPG70 ») ou par l'extrait PAT0014 (correspondant à un extrait selon l'invention) ou dans des fibroblastes NHDFs irradiés aux UVA, après 6h de récupération. Les fractions STL 964, STL 965, STL 967 et STL 1654 étant préparées dans du DMSO, leur fraction de contrôle est « CTL DMSO ». De même, l'extrait PAT0014 est préparé dans du DPG à 70%, sa fraction de contrôle correspond donc à « CTL DPG70 ». Les résultats sont exprimés en pourcentage relatif par rapport aux contrôles respectifs. L'analyse statistique effectuée est basée sur un test t-student comparant individuellement chaque traitement à son contrôle (* : p<0,05 ; ** : 0,001<p<0,01 et *** : p<0,001). Les valeurs p < 0,05 ont été considérées comme significatives, les valeurs 0,001 < p < 0,01 comme hautement significatives et les valeurs p < 0,001 comme très hautement significatives.

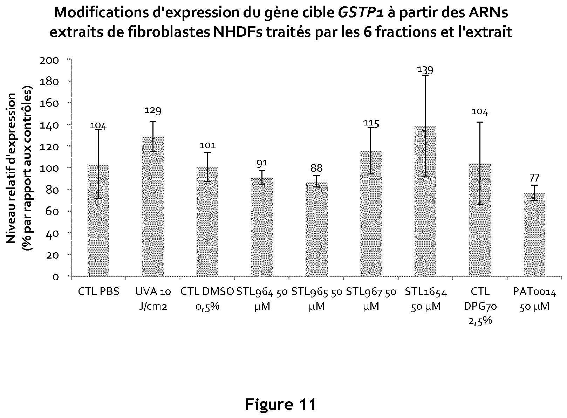

Figure 11. Niveau d'expression du gène GSTP1 dans des fibroblastes NHDFs traités pendant 24h par du PBS (contrôle « CTL PBS »), du DMSO (contrôle « CTL DMSO »), les fractions STL964 (comprenant majoritairement du 3,4-DCQ), STL 965 (comprenant majoritairement du 3,5-DCQ), STL 967 (comprenant majoritairement du 4,5-DCQ) et STL1654 (comprenant majoritairement des esters de DPG de DCQ), du DPG à 70% dans de l'eau v/v (contrôle « CTL DPG70 ») ou par l'extrait PAT0014 (correspondant à un extrait selon l'invention) ou dans des fibroblastes NHDFs irradiés aux UVA, après 6h de récupération. Les fractions STL 964, STL 965, STL 967 et STL 1654 étant préparées dans du DMSO, leur fraction de contrôle est « CTL DMSO ». De même, l'extrait PAT0014 est préparé dans du DPG à 70%, sa fraction de contrôle correspond donc à « CTL DPG70 ». Les résultats sont exprimés en pourcentage relatif par rapport aux contrôles respectifs. L'analyse statistique effectuée est basée sur un test t-student comparant individuellement chaque traitement à son contrôle (* : p<0,05 ; ** : 0,001<p<0,01 et *** : p<0,001). Les valeurs p < 0,05 ont été considérées comme significatives, les valeurs 0,001 < p < 0,01 comme hautement significatives et les valeurs p < 0,001 comme très hautement significatives.

### DESCRIPTION DETAILLEE DE L'INVENTION

### Définitions

Par « glycol » on entend un diol dans lequel les deux groupes hydroxyle sont portés par des carbones différents. Dans la présente description, le terme « glycol » désigne du glycol pur à 100% ou en solution, notamment en solution aqueuse, dans laquelle ledit glycol est présent à hauteur de 10% à 99%, plus particulièrement entre 40% et 90%, et encore plus particulièrement entre 50% et 75%.
Selon un aspect particulier, le glycol pur ou en solution, notamment aqueuse, est caractérisé par un pH acide, notamment lorsqu'il est utilisé lors d'une étape d'extraction solide/liquide. Selon un aspect particulier, ledit glycol utilisé lors d'une étape d'extraction solide/liquide est caractérisé par un pH compris entre 0,8 et 5, de préférence entre 0,8 et 3, plus préférentiellement entre 0,8 et 2,2. Selon un autre aspect particulier, un extrait végétal selon l'invention est caractérisé par un pH compris entre 2,5 et 5, de préférence entre 2,9 et 4,4.
Plus particulièrement, ledit glycol est choisi dans le groupe suivant : le dipropylène glycol, le propane 1,3 diol, le propane 1, 2 diol et le butylène glycol.

Par « dipropylène glycol » (abrégé en « DPG » dans toute la présente description), on entend un mélange des quatre composés chimiques isomères suivants : le 1-1'-oxydipropan-2-ol de formule (VIa), le 2-(2-hydroxypropoxy)propan-1-ol de formule (VIb), le 2-2'-oxydipropan-1-ol de formule (VIc) et le 1,1' oxy-dipropan-3-ol de formule (VId):

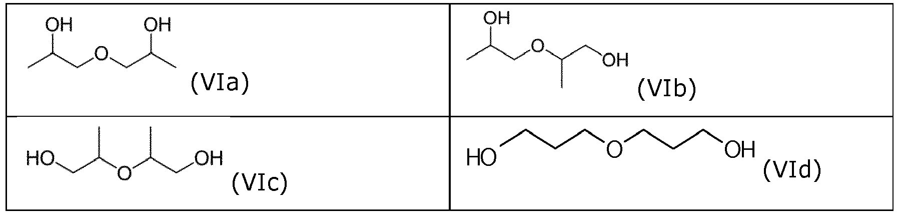

Selon la présente invention, les composés de formules (VIa), (VIb), (VIc) et (VId), telles que décrites ci-dessus, correspondent aux différents radicaux issus des quatre isomères du dipropylène glycol. Plus particulièrement, le radical de formule (IIa) est issu de l'isomère de formule (VIa), les radicaux de formules respectives (IIb) et (IIc) sont issus du même isomère de formule (VIb) et diffèrent par la position de l'hydroxyle qui se greffe au composé de formule générale (I), le radical de formule (IId) est issu de l'isomère de formule (VIc) et le radical de formule (IIe) est issu de l'isomère de formule (VId).

Par « propanediol » on entend le propane 1,3 diol et le propane 1,2 diol.
Le propane 1,3 diol, ou triméthylène glycol, répond à la formule (VII) suivante : 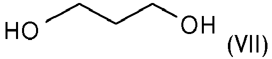 Ledit propane 1,3 diol peut être préparé par synthèse chimique selon des techniques connues de l'homme du métier et décrites dans la littérature, il est aussi disponible commercialement. Ledit propane 1,3 diol peut également être produit par la mise en œuvre d'un procédé de fermentation en conditions adaptées d'un organisme vivant, notamment une souche génétiquement modifiée *d'Escherichia coli.* Le propane 1,3 diol ainsi obtenu est désigné par le terme de « propane 1,3 diol biosourcé », un tel produit est produit puis purifié comme décrit notamment dans la demande internationale WO 2004/101479 et commercialisé sous la marque Zéméa®.

Selon la présente invention, le composé de formule (III) telle que décrite ci-dessus correspond au radical issu du propane 1,3 diol de formule (VII).

Le « propane 1,2 diol », ou propylène glycol, répond à la formule (VIII) suivante :

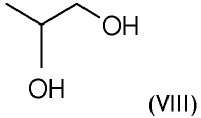

Selon la présente invention, les composés de formule (IVa) et (IVb) telles que décrites ci-dessus correspondent au radical issu du propane 1,2 diol de formule (VIII).

Par « butylène glycol » on entend le butane 1,2 diol de formule (IXa), le butane 1,3 diol de formule (IXb), le butane 2,3 diol de formule (IXc) et le butane 1,4 diol de formule (IXd), illustrées ci-dessous :

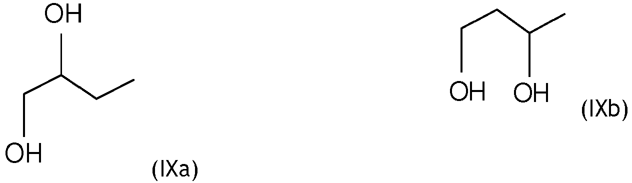

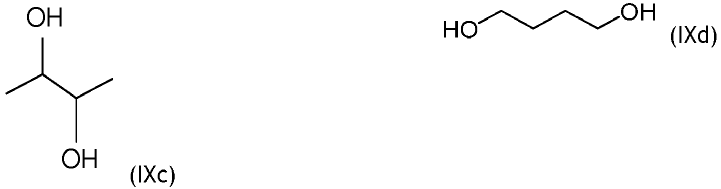

Selon la présente invention, les composés de formule (Va), (Vb), (Vc) et (Vd) telles que décrites ci-dessus correspondent aux radicaux issus des 4 isomères du butylène glycol : butane 1,2 diol (IXa), butane 1,3 diol (IXb), butane 2,3 diol (IXc) et butane 1,4 diol (IXd).

Par "acide dicaféoylquinique" (abrégé en « DCQ » dans toute la présente description), on entend un diester composé d'une molécule d'acide quinique dont deux des quatre fonctions alcools ont été estérifiées par une molécule d'acide caféique. Les DCQ sont donc des acides de formule générale (X) suivante : 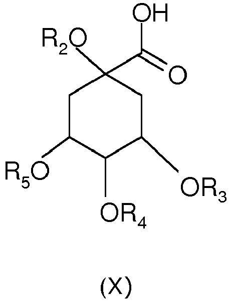 dans laquelle R₂, R₃, R₄ et R₅ sont tels que définis dans la formule (I) décrite ci-dessus (i.e. deux quelconques des radicaux R₂, R₃, R₄ et R₅ représentent un groupement caféoyle, les deux autres représentant un atome d'hydrogène).
Les différents isomères de DCQ sont donc des acides de formule générale (X), avec R₂, R₃, R₄ et R₅ tels que définis dans le Tableau 1 ci-dessous.

**Tableau 1. Structure des acides dicaféoylquiniques.**

| **Acides dicaféoylquiniques (DCQ)** | **R₂** | **R₃** | **R₄** | **R₅** |
|---|---|---|---|---|
| 1,3-dicaféoylquinique (1,3-DCQ) | Caféoyle | Caféoyle | H | H |
| 1,4-dicaféoylquinique (1,4-DCQ) | Caféoyle | H | Caféoyle | H |
| 1,5-dicaféoylquinique (1,5-DCQ) | Caféoyle | H | H | Caféoyle |
| 3,4-dicaféoylquinique (3,4-DCQ) également appelé acide isochlorogénique B | H | Caféoyle | Caféoyle | H |
| 3,5-dicaféoylquinique (3,5-DCQ) également appelé acide isochlorogénique A | H | Caféoyle | H | Caféoyle |
| 4,5-O-dicaféoylquinique (4,5-DCQ) également appelé acide isochlorogénique C | H | H | Caféoyle | Caféoyle |

Par « groupement caféoyle», on entend un radical de formule générale (XI), issu de l'acide caféique : 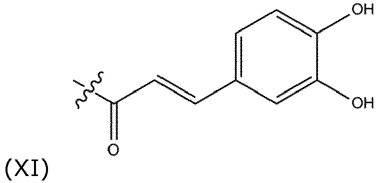 Par "acide chlorogénique", on entend l'ester simple de l'acide caféique et de l'acide quinique, également appelé trans-5-O-caféoyl-D-quinate, de formule (XII) : 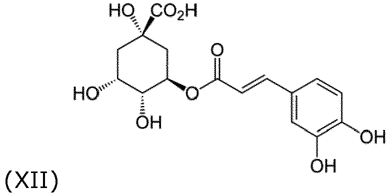 Par « plantes synthétisants des DCQ », on entend toute plante contenant naturellement, dans une ou plusieurs de ses parties, au moins un DCQ. Parmi les plantes connues pour contenir des DCQ, on peut citer celles des familles suivantes: Apiaceae (notamment Foeniculum vulgare), Aquifoliaceae (notamment les espèces Ilex), Araliaceae (notamment les espèces Cynara, les espèces Inula, les espèces Artemisia, Achillea millefolium, Taraxacum officinale, Echinacea angustifolia, Helianthus annuus, Smallanthus sonchifolius, Gynura divaricata, Echinops galalensis, Tanacetum vulgare, et Youngia japonica), Caprifoliaceae (notamment les espèces Lonicera et Knautia arvensis), Convolvulaceae (notamment les espèces Ipomoea, les espèces Convolvulus, Cressa cretica et Cuscuta japonica), Fabaceae, Rosaceae, Rubiaceae (notamment les espèces Coffea et Machaonia brasiliensis), et Solanaceae (notamment les espèces Solanum).

On entend par « partie » d'une plante toute partie constitutive d'une plante telle que les racines, la tige, les feuilles, le fruit, la peau, les graines ou le noyau.

Dans le cadre de la présente invention, on entend par « extraction solide/liquide » toute technique d'extraction par solvant qui consiste à extraire une espèce chimique se trouvant dans un solide et étant soluble dans ledit solvant. Parmi ces techniques d'extraction solide/liquide, on peut citer la macération, l'exsudation racinaire, l'infusion, la décoction, l'extraction par extracteurs de Soxhlet et de Kumagawa, l'extraction assistée par micro-onde, l'extraction assistée par ultrason, l'extraction par voie enzymatique, l'extraction par fluide supercritique (CO2 +DPG).

Dans le cadre de l'invention, on entend par « culture des plantes en conditions hors sol », tout mode de culture dans lequel les racines de la plante ne sont pas dans de la terre. Plus précisément, la culture hors sol est une culture dans laquelle les racines des plantes reposent dans un milieu reconstitué, détaché du sol. Ce milieu de culture est irrigué de façon régulière par des solutions nutritives appropriées à la plante cultivée.

Il existe différentes techniques de culture hors sol tels que les systèmes sans substrat qui nécessitent une solution nutritive enrichie en oxygène, et les systèmes avec substrat. Parmi les systèmes sans substrat, on peut citer l'aquiculture pour laquelle la solution nutritive est non circulante et est contenue dans un bac de culture, la Nutrient Film Technique (N.F.T.) pour laquelle la solution nutritive s'enrichit en oxygène dissous au cours de son déplacement par échange avec l'air, et l'aéroponie pour laquelle les racines des plantes ne sont en contact ni avec un milieu solide, ni même avec un milieu liquide : elles sont alimentées par un brouillard nutritif obtenu par brumisation (via un brumisateur) de la solution nutritive dans un milieu fermé. Parmi les systèmes avec substrat, on retrouve la subirrigation dans laquelle la solution nutritive pénètre dans le substrat au niveau de sa partie inférieure et la percolation dans laquelle la solution nutritive est distribuée par irrigation discontinue à la surface supérieure du système puis percole vers le bas du substrat. Le substrat, minéral ou organique, est neutre et inerte comme du sable, de l'argile ou de la laine de roche par exemple. Ce substrat peut être également d'origine industrielle.

Par « exsudation racinaire », on entend la récupération des métabolites contenus dans les racines des plantes au moyen d'un liquide mis en contact par percolation, aspersion ou immersion avec les racines encore accrochées à la plante vivante ou fraîchement coupées (depuis moins de 24 heures, et de préférence le plus tôt possible après coupage, idéalement juste après coupage). En particulier, l'exsudation racinaire peut être effectuée grâce à une étape de macération des racines des plantes, fraîchement coupées et/ou encore accrochées à la plante vivante, dans un solvant approprié et pendant une durée appropriée.

Dans le cadre de l'invention, par « exsudation racinaire dans un solvant », on entend une exsudation racinaire telle que définie ci-dessus pour laquelle le liquide mis en contact avec les racines est un solvant, ledit solvant est avantageusement un glycol choisi parmi : le dipropylène glycol, le propane 1,3 diol, le propane 1, 2 diol et le butylène glycol ou une solution contenant ledit glycol.

### Esters de glycol d'acides dicaféoylquiniques

La présente invention concerne donc des composés, esters de glycol d'acides dicaféoylquiniques, et notamment esters de dipropylène glycol d'acides dicaféoylquiniques et esters de propanediol d'acides dicaféoylquiniques, de formule générale (I) : 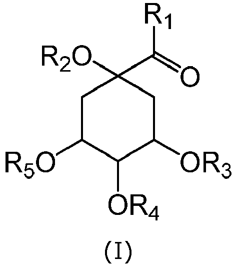 dans laquelle
- R₁ représente l'un des radicaux de formule (IIa), (IIb), (IIc), (IId), (IIe), (III), (IVa), (IVb), (Va), (Vb), (Vc) ou (Vd) suivante : 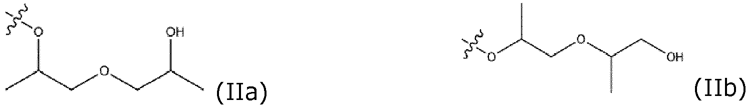 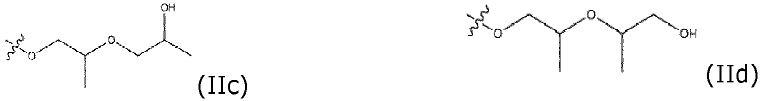 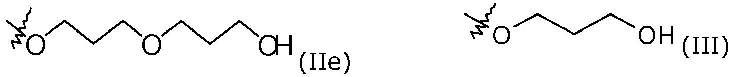 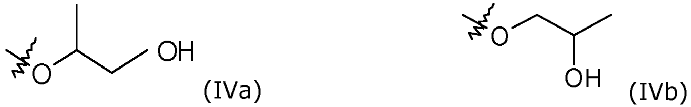 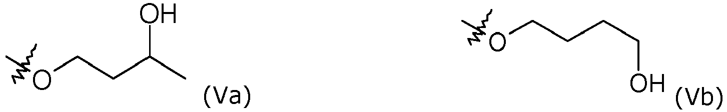 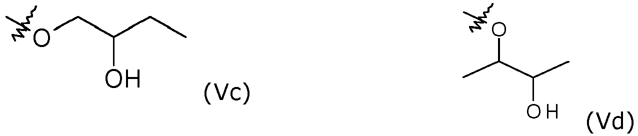
- Et deux quelconques des radicaux R₂, R₃, R₄ et R₅ représentent un groupement caféoyle, les deux autres représentant un atome d'hydrogène.

Parmi les composés de formule (I) selon la présente invention, on peut citer les esters de glycol, notamment les esters de DPG et les esters de propanediol de :
- L'acide 1,3-dicaféoylquinique,
- L'acide 1,4-dicaféoylquinique,
- L'acide 1,5-dicaféoylquinique,
- L'acide 3,4-dicaféoylquinique,
- L'acide 3,5-dicaféoylquinique, et
- L'acide 4,5-dicaféoylquinique.

Avantageusement, les composés de formule générale (I) selon l'invention sont caractérisés en ce que R₂ représente un atome d'hydrogène. Ainsi, parmi les composés avantageux de la présente invention on peut citer les esters de glycol, notamment les esters de DPG et esters de propanediol de :
- L'acide 3,4-dicaféoylquinique,
- L'acide 3,5-dicaféoylquinique, et
- L'acide 4,5-dicaféoylquinique.
Avantageusement, des composés de formule générale (I) selon l'invention sont caractérisés en ce que R₂ et R₄ représentent un atome d'hydrogène.
Des composés de formule générale (I) selon l'invention particulièrement avantageux sont ceux caractérisés en ce que R₂ et R₄ représentent un atome d'hydrogène et R₃ et R₅ représentent un groupement caféoyle, correspondant ainsi aux esters de glycol de l'acide 3,5-dicaféoylquinique, et notamment aux esters de DPG de l'acide 3,5-dicaféoylquinique (3,5-DCQ) et aux esters de propanediol de l'acide 3,5-dicaféoylquinique.
Encore plus avantageusement, selon un premier aspect, les composés esters de glycol selon l'invention sont des esters de DPG de 3,5-DCQ et sont sélectionnés parmi les molécules de formules respectives (Ia), (Ib), (Ic), (Id) et (Ie) suivantes :

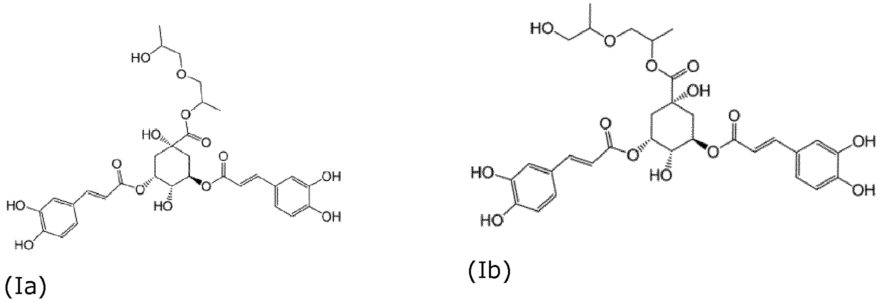

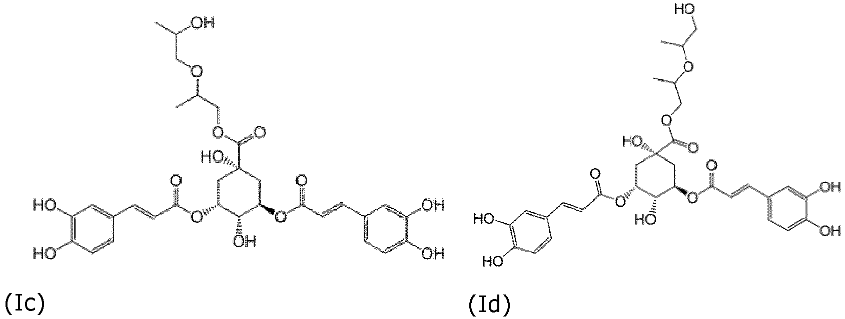

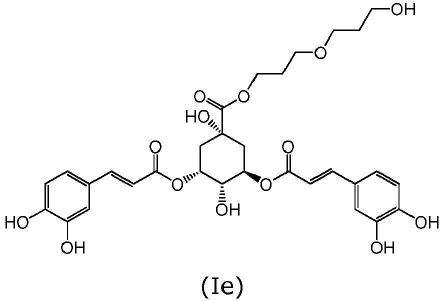

Selon cet aspect, les composés selon l'invention sont issus de l'estérification de l'acide 3,5-dicaféoylquinique et du dipropylène glycol (DPG).

Selon un deuxième aspect avantageux, le composé ester de propane 1,3 diol de 3,5-DCQ selon l'invention répond à la formule (If) suivante :

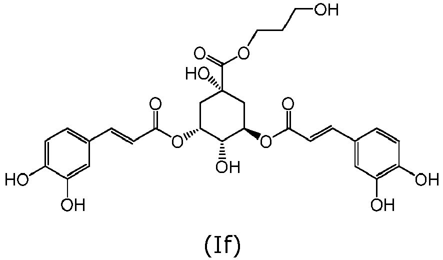

Selon cet aspect, les composés selon l'invention sont issus de l'estérification de l'acide 3,5-dicaféoylquinique et du propane 1,3 diol, notamment du propane 1,3 diol biosourcé.

Selon un troisième aspect avantageux, le composé ester de propylène glycol de 3,5-DCQ selon l'invention est sélectionné parmi les molécules de formules (Ig) et (Ih) suivantes :

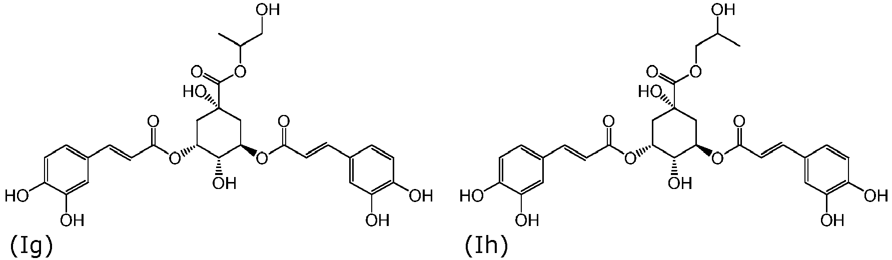

Selon cet aspect, les composés selon l'invention sont issus de l'estérification de l'acide 3,5-dicaféoylquinique et du propylène glycol.

Selon un quatrième aspect avantageux, le composé ester de butanediol de 3,5-DCQ selon l'invention est sélectionné parmi les molécules de formules (Ii), (Ij), (Ik) et (Il) suivantes :

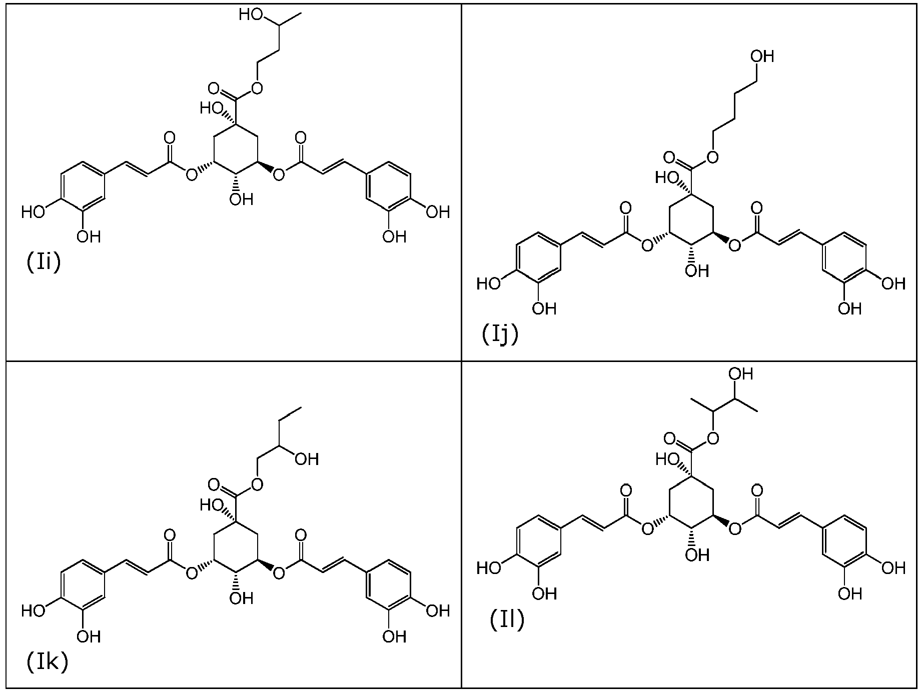

Selon cet aspect, les composés selon l'invention sont issus de l'estérification de l'acide 3,5-dicaféoylquinique et du butylène glycol.

Cette estérification peut être obtenue par synthèse chimique, notamment selon le schéma réactionnel ci-dessous pour le dipropylène glycol (DPG) et le propane 1,3 diol : 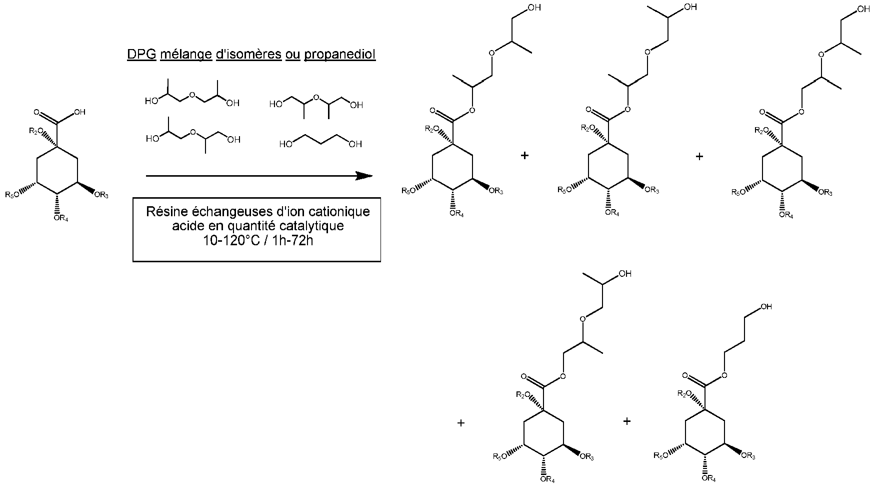 R₂, R₃, R₄ et R₅ sont tels que définis dans la formule (I) décrite ci-dessus (i.e. deux quelconques des radicaux R₂, R₃, R₄ et R₅ représentent un groupement caféoyle, les deux autres représentant un atome d'hydrogène).
Dans ce mode de réalisation, le(s) DCQ peut/peuvent être d'origine naturelle, les esters de glycol de DCQ, notamment les esters de DPG de DCQ ou les esters de propane 1,3 diol de DCQ, étant alors obtenus par hémisynthèse.
Dans un aspect préféré, les composés de la présente invention sont obtenus par extraction végétale de plantes synthétisants des DCQ, ou particulièrement riches en DCQ, dans du glycol ou dans une solution aqueuse de glycol, ledit glycol étant choisi parmi : le dipropylène glycol, le propane 1,3 diol, le propane 1,2 diol et le butylène glycol.

Par « extraction végétale » on entend tout procédé d'extraction connu de l'homme du métier, avantageusement une extraction solide/liquide d'une ou de plusieurs parties d'une plante synthétisant des DCQ dans du glycol ou dans une solution aqueuse de glycol, ledit glycol étant choisi parmi : le dipropylène glycol, le propane 1,3 diol, le propane 1,2 diol et le butylène glycol. A l'issue de l'extraction, des étapes d'extraction liquide/liquide, de purification, de concentration, de filtration clarifiante et/ou de décoloration peuvent avantageusement permettre d'obtenir les composés selon l'invention ou un extrait végétal concentré en composés selon l'invention. Toutes ces techniques sont décrites dans la littérature et connues de l'homme du métier.

### Extrait végétal comprenant au moins un des esters de glycol, notamment un ester de DPG du DCQ ou un ester de propane 1,3 diol du DCQ

Les inventeurs ont découvert que des extraits végétaux de plantes synthétisant des DCQ obtenus par extraction desdites plantes dans du glycol, ou dans une solution de glycol, notamment du dipropylène glycol ou du propane 1,3 diol, comprennent des composés selon l'invention et présentent un effet hormétique.

Dans un deuxième aspect, la présente invention concerne donc un extrait végétal comprenant au moins un composé de formule générale (I) selon la présente invention.
Plus particulièrement, la présente invention concerne un extrait végétal comprenant au moins un composé de formule générale (I) selon l'invention, ledit végétal étant sélectionné parmi une ou plusieurs partie(s) (notamment les racines) d'une plante synthétisant des DCQ telle que définie ci-dessus, notamment des espèces Ipomoea, et en particulier de l'espèce *Ipomoea batatas.*
La présente invention concerne donc notamment un extrait *d'Ipomoea batatas* comprenant au moins un composé selon l'invention, et en particulier un extrait racinaire *d'Ipomea batatas* comprenant au moins un composé selon l'invention.

En outre, du fait que les plantes utilisées pour l'obtention d'un extrait végétal selon l'invention sont de préférence particulièrement riches en DCQ et que l'estérification des DCQ à partir de glycol ou d'une solution aqueuse de glycol, notamment à partir de DPG ou de propane 1,3 diol, ou d'une solution aqueuse de DPG ou de propane 1,3 diol, peut dans certains modes de réalisation être partielle, l'extrait végétal selon l'invention comprenant au moins un composé selon l'invention peut également comprendre un acide dicaféoylquinique (DCQ) et/ou un acide chlorogénique.
Dans un mode de réalisation, la présente invention concerne donc un extrait végétal comprenant au moins un composé selon la présente invention, et un acide dicaféoylquinique et/ou un acide chlorogénique.
L'extrait végétal selon la présente invention peut notamment comprendre au moins un composé selon la présente invention et au moins l'un des composés suivants : l'acide 3,5-di-caféoylquinique, l'acide 3,4-dicaféoylquinique et l'acide 4,5-di-caféoylquinique.
Selon cet aspect, un extrait végétal selon l'invention est un extrait racinaire *d'Ipomoea batatas* comprenant au moins un composé selon l'invention, notamment un ester de glycol de DCQ, et comprenant une quantité d'ester de glycol de DCQ comprise entre 70 et 1400 mg/L, exprimés en mg d'ester ou d'acide par volume d'extrait végétal.
L'extrait végétal selon l'invention est avantageusement un extrait racinaire *d'Ipomoea batatas* comprenant au moins un composé selon l'invention et ayant la composition donnée dans le Tableau 2 suivant :

**Tableau 2. Composition d'un extrait racinaire d'Ipomoea batatas selon l'invention**

| **Composés présents** | **Teneur (mg/L)** |
|---|---|
| 3,4-DCQ / Acide isochlorogenique B | 50-150 |
| 3,5-DCQ / Acide Isochlorogenique A | 600-1200 |
| 4,5-DCQ / Acide Isochlorogenique C | 100-200 |
| Esters de DPG de 3,5-DCQ | 80-240 |

Selon un autre aspect, l'extrait végétal selon l'invention est avantageusement un extrait racinaire *d'Ipomoea batatas* comprenant au moins un composé selon l'invention et comprenant une quantité d'ester de propane 1,3 diol de 3,5-DCQ comprise entre 700 et 1400 mg/L.

Selon un aspect particulier, l'invention a pour objet un extrait végétal, notamment un extrait racinaire, plus particulièrement choisi parmi :
- Un extrait obtenu par exsudation racinaire dans un solvant,
- Un extrait obtenu par macération racinaire dans un solvant, et
- Un mélange d'au moins un extrait obtenu par exsudation racinaire dans un premier solvant et d'au moins un extrait obtenu par macération racinaire dans un deuxième solvant ;
Conformément à l'invention, ledit solvant est soit du glycol pur, soit une solution aqueuse de glycol; dans le cas d'un mélange d'extraits racinaires, lesdits premier et deuxième solvants peuvent être identiques ou différents; conformément à l'invention les solvants utilisés pour l'exsudation et la macération racinaires sont choisis parmi : le dipropylène glycol, le propane 1,2 diol, le propane 1,3 diol et le butylène glycol.

Selon un aspect particulier, l'invention a pour objet un extrait *d'Ipomoea batatas,* notamment un extrait racinaire *d'Ipomoea batatas,* plus particulièrement choisi parmi :
- Un extrait *d'Ipomoea batatas* obtenu par exsudation racinaire dans un solvant, de préférence le dipropylène glycol, l'étape d'exsudation racinaire étant de préférence réalisée sur des racines attachées à la plante,
- Un extrait *d'Ipomoea batatas* obtenu par macération racinaire dans un solvant, de préférence le dipropylène glycol, l'étape de macération racinaire étant réalisée de préférence sur des racines coupées et éventuellement séchées,
- Un extrait *d'Ipomoea batatas* obtenu par macération racinaire dans un solvant, de préférence le propane diol, plus préférentiellement le propane 1,3 diol, l'étape de macération racinaire étant réalisée de préférence sur des racines coupées et éventuellement séchées, et
- Un mélange comprenant un extrait *d'Ipomoea batatas* obtenu par exsudation racinaire dans le dipropylène glycol et un extrait *d'Ipomoea batatas* obtenu par macération racinaire dans le dipropylène glycol, et éventuellement un extrait *d'Ipomoea batatas* obtenu par macération racinaire dans le propane 1,3 diol.

### Procédé de préparation d'un extrait végétal selon l'invention et extrait susceptible d'être obtenu par ledit procédé

L'extrait végétal selon l'invention peut être obtenu par extraction solide/liquide de plantes synthétisant des DCQ dans du glycol ou une solution aqueuse de glycol, notamment du dipropylène glycol ou du propane 1,3 diol en tant que solvant, selon différents protocoles connus de l'homme du métier.

Selon un troisième aspect, la présente invention concerne donc un procédé de préparation d'un extrait végétal selon l'invention.

Ledit procédé selon l'invention comprend avantageusement une étape d'extraction solide/liquide d'une plante ou d'au moins une partie (notamment les racines) d'une plante synthétisant des DCQ (notamment *d'Ipomoea batatas*) dans du glycol, notamment du dipropylène glycol ou du propane 1,3 diol.

Avantageusement, l'extrait végétal selon l'invention est préparé selon un procédé de préparation comprenant les étapes suivantes :
a) Culture de plantes synthétisant au moins un acide dicaféoylquinique, et notamment *d'Ipomoea batatas*
b) Extraction solide/liquide des plantes issues de l'étape a) dans un solvant choisi parmi : le dipropylène glycol, le propane 1,3 diol, le propane 1,2 diol et le butylène glycol.

Selon un aspect particulier du procédé selon l'invention, l'étape d'extraction solide/liquide est réalisée par la mise en œuvre d'un procédé choisi parmi : une exsudation racinaire et une macération racinaire.

De préférence, dans un procédé selon l'invention, l'exsudation racinaire est réalisée sur des racines encore attachées à la plante ou fraîchement coupées. Selon un autre aspect préféré, la macération racinaire est réalisée sur des racines coupées et éventuellement séchées. Le séchage des racines peut être réalisé par la mise en œuvre de tout procédé de séchage adapté, connu de l'homme du métier, et notamment en plaçant les racines à une température comprise entre 40°C et 60°C pendant 4 heures, de préférence dans un environnement sec. Le séchage des racines peut notamment être réalisé dans une étuve ventilée.

Plus avantageusement, l'extrait végétal comprenant au moins un composé selon l'invention est préparé selon le procédé comprenant les étapes suivantes :
a) Culture de plantes synthétisant au moins un acide dicaféoylquinique, et en particulier de plantes *d'Ipomoea batatas,* en conditions hors sol,
b) Extraction solide/liquide par exsudation racinaire des plantes issues de l'étape a) dans du glycol pur ou une solution de glycol, notamment du dipropylène glycol ou du propane 1,3 diol, et
c) Eventuellement, récupération de l'extrait racinaire obtenu à l'étape b).

Avantageusement, la présente invention concerne donc un procédé permettant la préparation d'un extrait *d'Ipomoea batatas* comprenant au moins un composé selon l'invention et comprenant :
- la culture *d'Ipomoea batatas* en conditions hors sol,
- l'extraction solide/liquide *d'Ipomoea batatas* issues de l'étape de culture, par exsudation racinaire dans du glycol, notamment du dipropylène glycol ou une solution de dipropylène glycol,
- éventuellement l'extraction solide/liquide de racines *d'Ipomoea batatas* issues de l'étape de culture, fraîchement coupées et éventuellement séchées, par exsudation racinaire dans du glycol, notamment du dipropylène glycol, et
- éventuellement, la récupération de l'extrait, ou des extraits, obtenu(s) lors de l'étape d'extraction.

Selon un autre aspect encore plus particulier d'un procédé selon l'invention :
- l'extraction solide/liquide par exsudation racinaire est mise en œuvre alors que lesdites racines sont encore attachées à la plante, dans du dipropylène glycol, ou une solution de dipropylène glycol, dont le pH est compris entre 1,6 et 2,2 pendant 15 à 45 minutes, de préférence pendant 30 minutes,
   - ladite extraction est suivie de la coupure des racines, puis de l'extraction solide/liquide par macération desdites racines coupées, dans du dipropylène glycol, ou une solution de dipropylène glycol, dont le pH est compris entre 1,6 et 2,2 pendant 48 heures,
   - les extraits obtenus sont ensuite récupérés, mélangés, puis concentrés, et le solvant est ajusté entre 65% et 75% de DPG et un pH compris entre 2,9 et 3,1.

Selon un autre aspect avantageux d'un procédé selon l'invention, la culture de plantes synthétisant au moins un acide dicaféoylquinique, et notamment *d'Ipomoea batatas,* est suivie de la section des racines, et éventuellement du séchage des racines, l'extraction solide/liquide est mise en œuvre par macération dans un solvant choisi parmi : le dipropylène glycol, le propane 1,3 diol, le propane 1,2 diol et le butylène glycol.

Selon un aspect encore plus particulier, l'invention a pour objet un procédé comprenant la culture *d'Ipomoea batatas* en conditions hors sol, la section puis le séchage des racines, puis une étape de macération des racines coupées et séchées dans du propane 1,3 diol dont le pH est compris entre 0,8 et 1 pendant 2 à 8 jours, suivie de la récupération de l'extrait obtenu. La teneur en solvant est ajustée entre 50% et 60%, par ajout d'eau. Le pH de l'extrait est ensuite ajusté entre 4 et 4,4.

L'invention a également pour objet un extrait végétal susceptible d'être obtenu par un procédé selon l'invention.

### Applications

### Compositions cosmétiques

Pour être qualifiée d'hormétine, une substance doit induire une réponse cellulaire défensive notamment par la modification de l'expression de certains gènes, appelés vitagènes (S. I. Rattan. Ann. N. Y. Acad. Sci. 1998, 854: 54-60)*,* dans lesquels on retrouve particulièrement le facteur de transcription Nrf-2 et les gènes induits ou inhibés par ce facteur de transcription et notamment les gènes codants pour l'Hème-Oxygénase 1 (D. Gems, L. Partridge. Cell Metabolism, 2008, 7: 200-204)*,* l'HSP70, la NAD(P)H, la glutamate cystéine ligase, la glutathion S transférase pi, l'UDP-glucuronosyltransférase 1A6 et l'oxyde nitrique synthase.
Les inventeurs ont découvert que les composés selon l'invention, ou l'extrait végétal comprenant les composés selon l'invention, peuvent être qualifiés d'agents hormétiques et peuvent ainsi être utilisés pour atténuer ou retarder l'apparition des signes du vieillissement intrinsèque ou extrinsèque de la peau.

La présente invention concerne donc une composition cosmétique comprenant en tant qu'agent actif au moins un composé selon l'invention ou un extrait selon l'invention et avantageusement un excipient cosmétiquement acceptable.

Les modes d'administration, les posologies et les formes galéniques optimales des compositions cosmétiques selon l'invention peuvent être déterminés selon les critères généralement pris en compte dans l'établissement d'un traitement cosmétique adapté à un sujet comme par exemple le type de peau. En fonction du type d'administration souhaitée, la composition cosmétique selon l'invention peut en outre comprendre au moins un excipient cosmétiquement acceptable. La composition cosmétique selon la présente invention peut en outre comprendre au moins un adjuvant cosmétiquement connu de l'homme du métier, choisi parmi les épaississants, les conservateurs, les parfums, les colorants, des filtres chimiques ou minéraux, les agents hydratants, les eaux thermales, etc.

Avantageusement, la composition cosmétique comprend au moins un composé de formule générale (I) selon l'invention ou un extrait selon l'invention en une quantité comprise entre 0,01 et 10%, en particulier entre 0,05 et 5%, plus particulièrement entre 0,1 et 2%, en poids par rapport au poids total de la composition.

La présente invention concerne donc une composition cosmétique comprenant en tant qu'agent actif au moins un composé selon l'invention ou un extrait selon l'invention et avantageusement un excipient cosmétiquement acceptable.

La composition cosmétique selon l'invention peut en outre comprendre d'autres agents cosmétiquement actifs, tels que d'autres agents anti-âge ; ou bien des agents hydratants ; des agents ayant une activité calmante, apaisante ou relaxante ; des agents stimulant la microcirculation cutanée ; des agents sébo-régulateurs pour le soin des peaux grasses ; des agents nettoyant ou purifiant ; des agents anti-radicalaires ; des agents anti-inflammatoires ; des filtres solaires chimiques ou minéraux, etc.

L'excipient cosmétiquement acceptable peut être choisi parmi les polymères, les composés siliconés, les agents tensioactifs, les agents de rhéologie, les agents humectants, les agents de pénétration, les composants huileux, les cires, les émulsifiants, les agents filmogènes, les parfums, les électrolytes, les ajusteurs de pH, les agents antioxydants, les conservateurs, les colorants, les nacres, les pigments et leurs mélanges.

La composition cosmétique selon l'invention est avantageusement destinée à une application topique. Elle peut notamment se présenter sous la forme d'une crème, d'un lait, d'une lotion, d'un gel, d'un sérum, d'un spray, d'une mousse, d'une solution, d'une pommade, d'une émulsion, d'un patch ou d'un masque.

La composition cosmétique selon l'invention est destinée à retarder ou réparer l'apparition des signes du vieillissement (intrinsèque ou extrinsèque), et notamment du vieillissement extrinsèque. Avantageusement, elle peut notamment être destinée à empêcher ou inhiber la déstructuration de l'épiderme, la perte de fermeté ou d'élasticité de la peau, l'apparition des rides, de rougeurs, ou de taches de pigmentation de la peau. Elle permet en effet d'induire une réponse protectrice de la peau contre les agressions extérieures (exposition au soleil, à la lumière, ou aux UV ; stress ; malnutrition), la rendant ainsi moins sensible à ces agressions. De plus, la composition cosmétique permet également de restructurer l'épiderme, de raffermir la peau, et/ou de favoriser l'atténuation ou la résorption des rides.

L'invention a également pour objet un composé selon l'invention, un extrait selon l'invention ou une composition cosmétique selon l'invention, pour son utilisation pour prévenir le vieillissement cutané, pour atténuer les effets du vieillissement cutané, ou retarder l'apparition desdits effets, en particulier pour réduire et/ou retarder la formation de rougeurs, la formation des rides, la perte de fermeté de la peau, la perte de l'élasticité de la peau et la formation de taches de pigmentation de la peau, notamment les taches de pigmentation photo-induites et/ou les taches de sénescence.

L'invention concerne également l'utilisation en tant qu'agent actif d'un composé de formule (I) selon l'invention, ou d'un extrait selon l'invention, dans une composition cosmétique ou pour la préparation d'une composition cosmétique, pour prévenir le vieillissement cutané, pour atténuer les effets du vieillissement cutané, ou retarder l'apparition desdits effets, en particulier pour réduire et/ou retarder la formation de rougeurs, la formation des rides, la perte de fermeté de la peau, la perte de l'élasticité de la peau et la formation de taches de pigmentation de la peau, notamment les taches de pigmentation photo-induites et/ou les taches de sénescence.

Selon cet aspect, un composé, un extrait ou une composition cosmétique selon l'invention est utilisé(e) comme agent cosmétique pour prévenir le vieillissement cutané, atténuer les effets du vieillissement cutané, ou retarder l'apparition desdits effets.

L'invention concerne également un composé de formule (I) selon l'invention, ou d'un extrait selon l'invention, ou une composition cosmétique selon l'invention, pour prévenir le vieillissement cutané, pour atténuer les effets du vieillissement cutané, ou retarder l'apparition desdits effets, en particulier pour réduire et/ou retarder la formation de rougeurs, la formation des rides, la perte de fermeté de la peau, la perte de l'élasticité de la peau et la formation de taches de pigmentation de la peau, notamment les taches de pigmentation photo-induites et/ou les taches de sénescence.

L'invention concerne en outre une méthode de soin cosmétique de la peau visant à prévenir ou retarder l'apparition des effets du vieillissement (intrinsèque ou extrinsèque) de la peau, caractérisée en ce qu'elle comprend l'application sur au moins une partie de la peau du corps ou du visage d'une composition cosmétique selon l'invention.
Avantageusement, dans la méthode selon l'invention, la composition cosmétique est appliquée chez un sujet en ayant besoin, notamment en prévision de ou consécutivement à une exposition unique ou répétée de la peau à un stress oxydant.

Les exemples qui suivent visent à illustrer la présente invention.

### EXEMPLES

### Exemple 1 : Préparation et effet d'extraits racinaires d'Ipomoea batatas dans du DPG sur des fibroblastes - Etude en puces à ADN "anti-âge"

Afin d'analyser la possibilité qu'un extrait selon l'invention possède une action anti-âge, les variations d'expression de 92 gènes impliqués dans le vieillissement cellulaire et cutané induites par deux extraits racinaires *d'Ipomoea batatas* chez des fibroblastes ont été mesurées par qRT-PCR, et comparées aux variations induites par le TGF-β1 (contrôle positif), ce dernier ayant des effets bien documentés sur certains des gènes analysés.

### 1.1. Matériels et méthodes

### Extrait

Deux extraits racinaires *d'Ipomoea batatas,* possédant respectivement des concentrations en 3,5-DCQ de 1g/L (extrait STR-0003-P25-P0001) et 2g/L (STR-0003-P12-P0002), ont été préparés par exsudation racinaire dans du DPG de plante *d'Ipomoea batatas* cultivées hors sol. Le 3,5-DCQ est le composé majoritaire de ces deux extraits (12-15% en masse de l'extrait sec total). Ces deux extraits comprennent également dans une moindre proportion de l'acide chlorogénique, des esters de DPG de DCQ et d'autres dérivés d'esters d'acide caféique et leurs isomères. Il s'agit donc d'un mélange de composés de formule (I), dans lequel le 3,5-DCQ est présent de façon très majoritaire.

### Culture Cellulaire

L'étude a été réalisée sur des fibroblastes de derme humains NHDFs (ATCC, CRL-2522, origine : prépuce) cultivés en monocouche en milieu DMEM (Invitrogen, 31885-049) contenant 10% de sérum de veau foetal (Invitrogen, 10270-106) et des antibiotiques (Pénicilline/Streptomycine, Invitrogen, 15140-122). Ces cellules ont été maintenues dans une atmosphère humide à 37°C contenant 5% de CO2.

### Détermination des concentrations d'analyse des 2 actifs par une étude de cvtotoxicité

Afin de déterminer la concentration d'analyse optimale pour l'extrait STR-0003-P25-P0001 et pour l'extrait STR-0003-P12-P0002, une expérience préliminaire a été réalisée sur fibroblastes NHDFs. Cette étude a consisté à évaluer la viabilité des cellules au MTS (3-(4,5-dimethythiazol-2-yl)-5-(3-carboxy-methoxyphenyl)-2-(4-sulfophenyl)-2H-tetrazolium) (Promega,G3581) après 24 heures de traitement par les produits. Les cellules NHDFs ont été ensemencées en plaque 24-puits, 24h avant le traitement avec différentes concentrations des produits à tester. Au terme de cette expérience, une concentration non cytotoxique a été définie afin de procéder à la mesure des modifications d'expression des gènes cibles.

Les concentrations suivantes ont été testées :
- Extrait STR-0003-P25-P0001 : 0.5 µg/ml, 5µg/ml, 25 µg/m, 50 µg/ml, 75 µg/ml, 100 µg/ml, 150 µg/ml, 200 µg/ml, et 250 µg/ml ;
- Extrait STR-0003-P12-P0002 : 25 µg/ml, 50 µg/ml, 75 µg/ml, 100 µg/ml, 200 µg/ml.

### Analyse des modifications d'expression génique

### Extraction de l'ARN total

L'extraction des ARNs totaux a été réalisée à l'aide du kit RNeasy Mini (Qiagen, 74106). Après 24h de traitement, les cellules ont été rincées au PBS et lysées dans le tampon de lyse (des triples de culture ont été réalisés pour chaque condition). L'extraction et la purification des ARNs ont été réalisées selon les instructions du fournisseur. Les ARNs totaux ont ensuite été conservés à -80°C.

### Qualification des ARNs par spectrophotométrie et électrophorèse capillaire

La concentration des ARNs totaux a été déterminée par mesure spectrophotomètrique. La qualité et l'intégrité des ARNs a ensuite été vérifiée par électrophorèse capillaire (plate-forme *Agilent Bioanalyzer 2100).*
- Quantification des ARN par mesure spectrophotométrique : un aliquot de chaque ARN a été dilué dans de l'eau RNAse-free et sa concentration a été déterminée à l'aide d'un spectrophotomètre *Ultrospec 1100 Pro* (Amersham).
- Intégrité des ARN par électrophorèse capillaire sur Bioanalyseur Agilent : l'intégrité de l'ARN total a été évaluée par la visualisation des pics d'électrophorèse correspondant aux ARNs ribosomiaux. Pour les ARN totaux d'eucaryotes supérieurs, la taille des bandes ribosomales doit être de 1,9 kb pour le 18S-ARN et de 4,7 kb pour le 28S-ARN. L'intensité de la bande supérieure doit représenter environ deux fois l'intensité de la bande inférieure. Des petites bandes diffuses représentant des ARNs de poids moléculaire plus faible (ARNt et l'ARN ribosomique 5S) peuvent être présentes. Lorsque l'ARN est dégradé, on observe un étalement des bandes de l'ARN ribosomal ainsi qu'un bruit de fond des ARN de poids moléculaire plus élevé.

### Synthèse des ADNs complémentaires ou ADNc

Les transcriptions inverses (RT) ont été réalisées à l'aide du kit « *High Capacity RNA-to-cDNA Kit* » (Applied Biosystems, 4387406). Pour la synthèse des ADNc, un mix a été préparé selon les instructions du fournisseur, avec 2 µg d'ARN total, le tampon *ad hoc* fourni dans le kit et l'enzyme transcriptase inverse. Cette réaction a été réalisée à 37°C pendant 1 heure, ensuite 5 minutes à 95°C et finalement les échantillons d'ADNc sont mis sur glace et stockés à -20°C.

### Préparation des cartes microfluidiques Taqman, réalisation de la PCR quantitative et analyse des Ct

Les mélanges réactionnels pour PCR sur cartes microfluidiques TaqMan, produites à façon par *Applied Biosystems,* ont été préparés en suivant les instructions détaillées du guide *Applied Biosystems Micro Fluidic Card Getting Started Guide.* En résumé, 100ng d'ADNc ont été ajoutés à un mélange spécifique pour PCR avant d'être injectés dans la carte et dispersés par capillarité. Après avoir centrifugé la carte, celle-ci a été scellée avant la réalisation de la PCR quantitative et l'analyse avec le système 7900HT d'Applied Biosystems, à l'aide du software *ABI PRISM*® *7900 Sequence Detection System, SDS2.1.* Ce programme a permis de mesurer les cycles seuil Ct (*Cycle threshold =* cycle seuil de détection à partir duquel la quantité d'ADN est telle que le signal se distingue significativement du bruit de fond). L'analyse des niveaux d'expression a été réalisée à l'aide du logiciel *Data Assist* de chez *Applied Biosystems.* La méthode utilisée pour calculer la modification d'expression génique est basée sur la valeur du Ct.

### 1.2. Résultats

### Détermination de la concentration d'analyse des extraits par une étude de cytotoxicité

Une étude de cytotoxicité de l'extrait a été réalisée sur des fibroblastes de derme.

Le solvant présent dans les produits a été testé en parallèle. Pour chaque concentration testée de l'extrait, le solvant STR-DPG70 a été testé seul à une concentration équivalente à celle présente dans l'extrait.

Le SDS à 0,08% a été utilisé comme contrôle positif de cytotoxicité afin de valider l'expérience.

Les résultats sont illustrés sur les Figures 1 et 2, et montrent que l'extrait STR-0003-P25-P0001 testé à 25, 5 et 0,5 µg/ml et l'extrait STR-0003-P12-P0002 testé à 25, 50 et 75 µg/ml n'induisent aucune cytotoxicité.

Cependant, nous observons une perturbation morphologique (présence de quelques cellules flottantes) lorsque les cellules sont traitées avec 75 µg/ml de l'actif.

Sur la base de ces résultats, les concentrations suivantes ont été choisies pour la suite de l'étude : 25 µg/ml (Extrait STR-0003-P25-P0001) et 50 µg/ml (Extrait STR-0003-P12-P0002).

### Qualification des ARNs par électrophorèse capillaire

Les différentes populations d'ARN démontrent bien la présence de pics étroits, correspondant aux ARN ribosomiaux 18S et 28S, et un rapport équilibré entre les deux pics. L'absence de pics intermédiaires et étalés, caractéristiques de produits de dégradation des ARNs témoigne de l'intégrité des différentes populations (données non montrées).

La qualité et l'intégrité des ARNs extraits étant démontrée, ceux-ci ont dès lors été utilisés pour la poursuite du protocole et engagés dans les réactions de synthèse des ADNs complémentaires.

### Analyse des modifications d'expression de gènes induites par les extraits STR-0003-P25-P0001 et STR-0003-P12-P0002 sur fibroblastes NHDFs

Les extraits STR-0003-P25-P0001 et STR-0003-P12-P0002 et le solvant (STR-DPG70) ont été appliqués sur des fibroblastes, durant 24 heures. Au terme de l'application, les populations d'ARN totaux ont été extraites et analysées par qRT-PCR à l'aide d'une carte TaqMan ciblant le vieillissement et la matrice extracellulaire. Comme attendu, le TGF-β, élément de référence (ou « contrôle positif interne ») a un effet sur un certain nombre de gènes et valide de ce fait le système d'essai.

Les résultats de l'extrait, exprimés sous forme de RQ, expression relative, sont repris dans le Tableau 3 et rapportés au traitement par le solvant STR-DPG70. Les expériences ont été réalisées en triple de culture. Les valeurs 0,01 <p< 0,05 ont été considérées comme significatives, les valeurs 0,001 <p< 0,01 comme hautement significatives et les valeurs p< 0,001 comme très hautement significatives.

**Tableau 3. Liste comparative des gènes modifiés au moins de manière significative par le traitement par l'extrait STR-0003-P25-P0001 à 25 µg/ml ou par l'extrait STR-0003-P12-P0002 à 50 µg/ml.**

| Nom des gènes exprimés | Symbole des gènes | STR-0003-P25-P0001 | | STR-0003-P12-P0002 | | Fonction générale |
|---|---|---|---|---|---|---|
| | | RQ | P-value | RQ | P-value | |
| **Heme oxygenase (decycling)1** | **HMOX1** | **7,5492** | **0,0043** | **12,9967** | **0,0237** | **Défense** |
| Caveolin 1 | CAV-1 | 0,8456 | 0,0412 | 0,6953 | 0,0351 | transduction du signal |
| Collagen, type III, alpha 1 | COL3A1 | 0,5397 | 6,00E-04 | 0,6831 | 0,0402 | communication cellulaire / adhésion focale / interaction ECM-récepteurs |
| Connective tissue growth factor | CTGF | 0,6982 | 0,0186 | 0,5252 | 0,0135 | Facteurs de croissance et cytokines |
| Fibulin 5 | FBLN5 | 0,6753 | 0,0252 | 0,7336 | 0,0265 | matrice extra cellulaire |
| Fibroblast growth factor | FGF7 | 0,5991 | 0,0428 | 0,4304 | 0,0016 | Croissance cellulaire |
| Glutathione peroxidase 1 | GPX1 | 0,3134 | 0,0091 | 0,3422 | 0,0032 | défense enzymatique antioxydante |
| Insulin-like growth factor 1 | IGF1 | 0,3232 | 4,00E-04 | 0,3089 | 0,0025 | Croissance cellulaire |
| Insulin-like growth factor binding protein 3 | IGFBP3 | 0,5961 | 0,0274 | 0,3927 | 0,003 | prolifération cellulaire /apoptose |
| Insulin-like growth factor binding protein 5 | IGFBP5 | 0,619 | 0,0138 | 0,5403 | 0,0072 | prolifération cellulaire /apoptose |
| Retinoblastoma 1 | RB1 | 0,8212 | 0,0197 | 0,6759 | 0,0232 | Cycle cellulaire |
| Thymidine kinase 1 | TK1 | 1,1985 | 0,0023 | 0,4939 | 0,0034 | métabolisme pyrimidine |
| **Heat shock 70KDa protein 1A** | **HSPA1A** | **1,8348** | **0,0281** | | | **défense** |
| **NAD(P)H dehydrogenase, quinone 1** | **NQO1** | **1,7295** | **0,0265** | | | **défense enzymatique antioxydante** |
| Activator protein 1 | JUN | 1,4691 | 0,0119 | | | Activité facteur de transcription |
| Proliferating cell nuclear antigen | PCNA | 0,8358 | 0,0189 | | | Réparation de l'ADN |
| Ataxia telangiectasia mutated | ATM | 0,8223 | 0 | | | Réparation/synthèse de l'ADN |
| Calnexin | CANX | 0,7974 | 0,0404 | | | Repliement et assemblage des protéines |
| Polymerase (RNA)II(DNA directed) polypeptide A | POLR2A | 0,7747 | 0,0095 | | | Gène de ménage |
| Fibrillin 1 | FBN1 | 0,771 | 0,0447 | | | matrice extracellulaire |
| BCL2-associated X protein | BAX | 0,7407 | 0,0199 | | | apoptose |
| Forkhead box 3 | FOXO3 | 0,7405 | 0,026 | | | Facteur de transcription |
| 8-oxoguanine DNA glycosylase | OGG1 | 0,6915 | 0,0139 | | | Réparation de l'ADN |
| Matrix metallopeptidase 2 | MMP2 | 0,6699 | 0,0305 | | | matrice extracellulaire |
| Syndecan 3 | SDC3 | 0,6693 | 0,0049 | | | Forme cellulaire, signalisation cellulaire |
| Sirtuin 2 | SIRT2 | 0,6607 | 0,0306 | | | Extinction de gène |
| Secreted protein, acidic, cysteine-rich (osteonectin) | SPARC | 0,6297 | 0,0156 | | | matrice extracellulaire |
| Collagen, type I, alpha 2 | COL1A2 | 0,6128 | 0,0097 | | | communication cellulaire / adhésion focale / interaction ECM-récepteurs |
| Elastin | ELN | 0,3824 | 0,05 | | | matrice extracellulaire |
| Growth arrest and DNA-damage-inducible | GADD45A | | | 1,2345 | 0,0343 | Réparation de l'ADN |
| Lysyl oxidase-like 1 | LOXL1 | | | 0,7837 | 0,0435 | matrice extracellulaire |
| Peroxiredoxin 5 | PRDX5 | | | 0,7794 | 0,0475 | défense |
| Transforming growth factor, beta 1 | TGFB1 | | | 0,6821 | 0,0281 | Prolifération cellulaire |
| Heat shock 27KDa protein 1A | HSPB1 | | | 0,5339 | 0,0023 | défense |
| V-Fos FBJ murine osteosarcoma viral oncogene | FOS | | | 0,4793 | 0,0088 | cycle cellulaire |
| Matrix metallopeptidase 3 | MMP3 | | | 0,4585 | 0,0395 | matrice extracellulaire |
| Transgelin | TAGLN | | | 0,364 | 4,00E-04 | structure cellulaire |

D'une manière générale, l'analyse du Tableau 3 montre que :
- Peu de gènes sont surexprimés en réponse aux extraits STR-0003-P25-P0001 et STR-0003-P12-P0002.
- Toutefois, le gène **HMOX** est largement surexprimé d'un facteur 7,5x et 13x en réponse aux deux extraits STR-0003-P25-P0001 et STR-0003-P12-P0002 respectivement. Cette surexpression peut être qualifiée de tout-à-fait remarquable. Dans une moindre mesure **HSPA1A** et **NQO1** sont également surexprimés d'un facteur 1,8x et d'un facteur 1,7x respectivement en réponse à l'extrait STR-0003-P25-P0001.
- Davantage de gènes sont réprimés.
   Nous avons ainsi mis en évidence la sous-expression des gènes **GPX1** et **IGF1** (plus de 3x, RQ < 0,33) par les deux extraits STR-0003-P25-P0001 et STR-0003-P12-P0002. On peut également noter la sous-expression de plus de 2x (RQ < 0,5) de **l'élastine** en réponse à l'extrait STR-0003-P25-P0001, et de la transgeline par l'extrait STR-0003-P12-P0002.

### 1.3. Discussion

### Surexpression des « vitagènes »

Les gènes **HMOX** et **HSPA1A** appartiennent à la famille des **vitagènes,** comprenant en outre le facteur de transcription Nrf-2 (HSP32), la protéine chaperone HSP60 et les gènes du système thiorédoxine. Ces gènes sont le plus souvent surexprimés en réponse à un stress, et jouent un rôle crucial dans la protection et le maintien de l'homéostasie cellulaire.

La protéine **HSP70** (codé par le gène **HSPA1A)** est une molécule chaperone (*heat shock protein 70*) qui inhibe l'aggrégation des protéines dénaturées, favorise leur renaturation (refolding) et contrôle des médiateurs essentiels de la machinerie apoptotique.

La surexpression des vitagènes **HO-1** ou **HSP70** suite à des stress cellulaires, en particulier de type radicalaires, est associée au concept d'hormésis, qui repose sur le fait qu'une molécule ou un traitement générant un stress de faible intensité, sans dommages cellulaires importants, permet d'induire une réponse protectrice et antioxydante afin d'assurer l'homéostasie de la cellule (face à des molécules prooxydantes, un stress thermique, des hormones, une restriction calorique, etc.), préparant et protégeant ainsi la cellule contre des stress ultérieurs de plus grande intensité.

### Surexpression du gène NQO1

Le gène **NQO1** code pour la **NAD(P)H déshydrogénase (quinone),** possédant une activité cytoplasmique *2-electron réductase* témoignant d'une action détoxifiante. NQO1 est surexprimée en réponse à des agents prooxydants, à des métaux lourds, aux UV ou encore à des radiations ionisantes). NQO1 stabilise la protéine p53 contrôlant diverses enzymes de réparation et médiateurs de l'apoptose. L'enzyme NQO1 favorise par réduction la formation des hydroquinones à partir des quinones, empêchant la production d'espèces radicalaires. NQO1 est sous le contrôle du facteur de transcription Nrf-2 contrôlant la voie de réponse au stress oxydant (Nguyen et al, Biol. Chem., 284 (20), 13291-13295, 2009).

Il a été démontré que la protéine NQO1 s'associe à la protéine HSP70 pour renforcer sa stabilité et son activité (Anwar et al., J. Biol. Chem., 277, 14060-14067, 2002). La surexpression de **NQO1** induite par STR-0003-P25-P0001 et STR-0003- P12-P0002 est donc en faveur d'une activité protectrice vis-à-vis d'un stress cellulaire.

### Sous-expression des gènes GPX1, IGF1, élastine et TAGLN

Le gène **GPX1** code pour la **glutathione peroxidase 1,** impliquée dans la détoxification de l'H2O2 produit de façon endogène par la respiration mitochondriale et la synthèse d'ATP (phosphorylation oxydative). Dans ce cas, la répression du gène n'est pas en corrélation avec une activité antioxydante des produits.

Le gène **IGF1** code pour une hormone de croissance, **l*'insulin growth factor 1***. La diminution d'IGF1 circulante et l'inactivation du gène IGF1 ont été associées à une augmentation de la longévité d'organismes modèles tels que le nématode C. *elegans* ou la souris.

Le gène **ELN** code pour la **tropoélastine,** qui est un des composants majeurs des fibres élastiques avec la fibrilline. La réticulation de la tropoélastine, catalysée par la lysyl oxydase, forme l'élastine, dont la diminution avec l'âge résulte dans la formation des rides.

Enfin, le gène **TAGLN** code pour la **transgéline,** encore appelée SM22, dont la surexpression est reconnue comme un biomarqueur robuste des fibroblastes sénescents.

### 1.4. Conclusion

L'induction des gènes HMOX, HSP70 et NQO1 suggère qu'un extrait selon l'invention ou la molécule 3,5-DCQ, qui est largement majoritaire dans les extraits STR-0003-P25-P0001 et STR-0003- P12-P0002, pourrait exercer un effet protecteur vis-à-vis de stress radicalaires endogènes liés au vieillissement chronologique, ou exogènes tels qu'induits par les UVA ou divers agents polluants, et ce par un mécanisme basé sur le principe d'hormesis.

### Exemple 2 : Cinétique d'induction des protéines codées par les gènes HMOX, HSP70 et NQO1 suite à l'application d'extraits racinaires d'Ipomoea batatas dans du DPG et confirmation de l'effet protecteur de l'exposition de fibroblastes à des extraits racinaires d'Ipomoea batatas dans du DPG vis-à-vis de stress oxydants ultérieurs aux UV.

Il a été montré dans l'Exemple 2 que la mise en contact de fibroblastes avec deux extraits racinaires *d'Ipomoea batatas* comprenant comme composé majoritaire le 3,5-DCQ, induit une surexpression de protéines impliquées dans le processus d'hormésis au niveau ARNm. Afin de confirmer l'induction d'une surexpression de ces protéines par ces extraits au niveau protéique, des analyses ont été réalisées par Western Blot.

### 2.1. Matériels et méthodes

### Extrait

Deux extraits racinaires *d'Ipomoea batatas,* possédant respectivement des concentrations en 3,5-DCQ de 1g/L (extrait STR-0003-P33-P0004) et 2g/L (STR-0003-P33-P0003), ont été préparés tels que décrits dans l'exemple 1. Le 3,5-DCQ est le composé majoritaire de ces deux extraits (12-15% en masse de l'extrait sec total). Ces deux extraits comprennent également dans une moindre proportion de l'acide chlorogénique et d'autres dérivés d'esters d'acide caféique et leurs isomères. Il s'agit donc d'un mélange de composés de formule (I), dans lequel le 3,5-DCQ est présent de façon très majoritaire.

Comme dans l'Exemple 1, l'extrait STR-0003-P33-P0004 a été utilisé à une concentration de 25 µg/ml, tandis que l'extrait STR-0003-P33-P0003 a été utilisé à une concentration de 50 µg/ml.

### Culture cellulaire

L'étude a été réalisée sur des fibroblastes de derme humains NHDFs (ATCC, CRL-2522, origine : prépuce) cultivés en monocouche en milieu DMEM (Invitrogen, 31885-049) contenant 10% de sérum de veau fœtal (Invitrogen, 10270-106) et des antibiotiques (Pénicilline/Streptomycine, Invitrogen, 15140-122). Ces cellules ont été maintenues dans une atmosphère humide à 37°C contenant 5% de CO₂.

### Détermination par Western Blot de la cinétique d'induction de HO-1/HSP32 au niveau protéique

Les fibroblastes en culture ont été mis en présence des actifs durant 6, 24, 48 ou 72h. Les concentrations choisies pour les 2 actifs (50 µg/ml pour l'extrait STR-0003-P33-P0003 et 25 µg/ml pour STR-0003-P33-P0004) ont été déterminées suite à l'étude des concentrations d'analyse par une étude de cytotoxicité décrite dans l'exemple 2.

Le contrôle positif de l'expérience induisant l'expression de la protéine HO-1 est le célastrol (Sigma, C0869) utilisé à 250 nM.

### Extraction des protéines

Les protéines ont été extraites directement après l'application des actifs, après 6, 24, 48 ou 72 heures de contact, en utilisant une solution de T-PER (Tissue Protein Extraction Reagent, Pierce, ref 78510) associée à un inhibiteur de phosphatases (PhosSTOP, Roche, ref 04 906 837 001) et un inhibiteur de protéases (Complete Protease inhibitor cocktail, Roche, ref 04 693 116 001).

Les lysats ont ensuite été vortexés et soniqués à froid avant d'être centrifugés afin d'éliminer les débris cellulaire. Les surnageants obtenus ont été congelés à -80°C.

### Dosage des protéines

Le contenu en protéines des lysats cellulaires a été quantifié par une méthode colorimétrique, en utilisant le 660nm Protein Assay Reagent (Pierce, ref 22662). Les densités optiques obtenues pour les échantillons et les solutions de référence (Pre-diluted Protein Assay standards : BSA Set, ref 23208, Thermo Scientific) ont été utilisées afin de calculer la concentration en protéines de chaque échantillon.

### Western Blot

Les échantillons ont été chargés sur des gels de polyacrylamide 10% (Invitrogen, ref NP0301BOX) à raison de 10µg de protéines par échantillon. La migration a été effectuée durant 1 heure à 200 Volt. L'étape de transfert sur une membrane en PVDF (Polyvinylidene fluoride membrane, Pall Corporation, ref P/N66543) a été réalisée sur glace, durant 1 heure à 100 Volt. Les membranes ont été saturées dans une solution de lait à 2% (ECL détection kit, Amersham, RPN2135). Ensuite, les membranes ont été incubées toute une nuit, à 4°C, avec l'anticorps spécifique de la protéine HO-1/HSP32 (BD, ref 610712). Après rinçage, les membranes ont été mises en présence de l'anticorps secondaire, couplé à la peroxydase (HRP). La visualisation des bandes a été réalisée avec le kit contenant les substrats chemoluminescents (ECL detection kit, Amersham, RPN2135) et des films d'autoradiographie (hyperfilm ECL, Amersham, 28-9068-36). La tubuline-α a été utilisée comme protéine de référence afin de normaliser les valeurs. La quantification de l'intensité des bandes a été réalisée grâce au logiciel ImageJ.

### Mise en évidence de l'effet protecteur des actifs vis-à-vis d'un stress radicalaire pro-oxydant : validation de l'hypothèse d'un effet hormétique des actifs

### Confirmation de l'induction d'un stress radicalaire par les actifs

Afin de valider l'hypothèse d'un effet hormétique, il a été vérifié au préalable que le seul traitement par les actifs induisait un stress radicalaire. Le test utilisé est basé sur l'utilisation de la sonde fluorescente H2DCFDA (2'-7'dichlorodihydrofluorescein diacetate, Fischer Scientific, #D399). La diffusion de la sonde dans la cellule permet le clivage de son groupement acétate par des estérases intra-cellulaires. La sonde peut dès lors être oxydée par des radicaux libres (dérivés réactifs de l'oxygène (ROS)) en DCF (2'-7'-dichlorofluorescein), un composé fluorescent. La production des radicaux libres peut donc être quantifiée par le niveau de fluorescence émise par la sonde oxydée. Pratiquement, les cellules ont été traitées 4 ou 6 heures par les actifs et au terme de ces traitements, les cellules ont été rincées puis mises en présence de la sonde H2DCFDA à 5 µM pendant 1 heure à 37°C, 5% CO2 afin de permettre la diffusion intra-cellulaire de celle-ci. Après rinçage, la fluorescence émise a été quantifiée (excitation : 485 nm ; émisssion : 520 nm).

Afin de fournir un résultat représentatif de la population cellulaire de chaque condition, un test de viabilité cellulaire (MTS) a été réalisé immédiatement après la quantification de la fluorescence émise. Les valeurs de fluorescence ont ainsi été normalisées par rapport aux viabilités cellulaires.

### Mise en évidence de l'effet protecteur des actifs vis-à-vis d'un stress radicalaire non cytotoxique aux UVA

L'effet protecteur des éléments d'essai vis-à-vis d'un stress non-cytotoxique aux UVA (10J/cm2) sur fibroblastes a également été évalué par un test basé sur l'utilisation de la sonde fluorescente H2DCFDA. Les données obtenues ont été normalisées par rapport à la viabilité cellulaire (test MTS).

Pratiquement, les cellules ont été traitées pendant différents temps (4h et 6h) avec les actifs. Les cellules ont ensuite été rincées et remises dans du milieu frais durant 16 heures supplémentaires. Au terme de ces 16 heures de récupération, les cellules ont été rincées au PBS puis mises en présence de la sonde H2DCFDA à 5 µM pendant 1 heure à 37°C, 5% CO2 afin de permettre la diffusion intra-cellulaire de celle-ci. Après un rinçage, les cellules ont été soumises à un stress aux UVA à raison de 10 J/cm2. Une condition non exposée a été réalisée en parallèle. La quantification de la fluorescence émise (excitation : 485 nm ; émission : 520 nm) a été effectuée immédiatement après la fin du stress UVA. Comme précédemment, un test de viabilité au MTS a été réalisé afin de normaliser la fluorescence par rapport à la viabilité cellulaire.

### 2.2. Résultat

### Détermination par Western Blot de la cinétique d'induction de HO1-1/HSP32 au niveau protéique

La Figure 3 montre l'analyse de l'expression de la protéine HSP32/HO-1 par des fibroblastes traités par des milieux contrôles négatifs (CTL DMSO et CTL), un produit contrôle positif (Célastrol) et par les extraits STR-0003-P33-P0004 (25 µg/ml) et STR-0003-P33-P0003 (50 µg/ml), après 6h, 24h, 48h ou 72h d'incubation respectivement.

Elles confirment que les extraits STR-0003-P33-P0004 et STR-0003-P33-P0003 induisent une surexpression de la protéine HSP32/HO-1 chez les fibroblastes, générant ainsi une réponse protectrice chez ces cellules, réponse susceptible de les protéger contre des stress ultérieurs.

### Mise en évidence de l'effet protecteur des actifs vis-à-vis d'un stress radicalaire pro-oxydant : validation de l'hypothèse d'un mécanisme de type hormétique.

### Confirmation de l'induction d'un stress radicalaire par les extraits étudiés après 4 et 6 heures de contact

Dans un premier temps, afin de démontrer que les extraits étudiés protègent les cellules d'un stress radicalaire, et ce en se comportant à la manière d'une hormétine, il faut mesurer la production d'espèces réactives de l'oxygène (ROS) au terme d'un contact de 4 et 6 heures avec ces extraits et démontrer qu'ils génèrent un stress non cytotoxique, en particulier un stress radicalaire.

Cette expérience a démontré que le seul traitement des fibroblastes par les actifs (pendant 4 ou 6 heures) induit bien un stress de type radicalaire. En parallèle, le traitement par un antioxydant neutralisant les radicaux libres par une réaction d'oxydo-réduction classique, à savoir le Trolox, un analogue soluble de la vitamine E (Sigma 238813), n'induit aucun stress radicalaire puisqu'il n'agit pas selon un mécanisme hormétique. Les résultats obtenus sont synthétisés dans la Figure 4. Les valeurs de fluorescence ont été normalisées par rapport à la viabilité cellulaire évaluée par un test MTS. Les résultats sont exprimés en pourcentages par rapport à la condition témoin (EtOH 0.1% pour le traitement au Trolox et contrôle non traité pour le traitement par les actifs).

### Mise en évidence de l'effet protecteur des actifs vis-à-vis d'un stress radicalaire non cytotoxique aux UVA.

Dans un second temps, afin de démontrer un effet protecteur lié à l'application des extraits durant 4 et 6 heures, face à un stress appliqué au terme d'une période de récupération de 16 heures.

Les résultats illustrés à la Figure 5 montrent qu'une incubation avec les actifs durant des temps courts (4 ou 6h) suivi de 16 heures de récupération protège les cellules contre la formation de radicaux libres induits par un stress non cytotoxique aux UVA. Cette cinétique a fait l'objet de mises au point préliminaires et a été dûment optimisée. Comme précédemment, les données de fluorescence ont été normalisées par rapport à la viabilité des cellules, évaluée par un test MTS après le stress aux UVA. Les résultats confirment l'hypothèse d'un effet protecteur induit par les actifs, consistant en une atténuation de la production de radicaux libres suite à une exposition aux UVA.

### 2.3. Conclusion

Au terme de ces études, les expériences réalisées démontrant une forte induction de la protéine HO-1 ainsi qu'un effet protecteur d'un contact des cellules avec les extraits, vis-à-vis des effets d'une exposition aux UVA, principale cause du photo-vieillissement cutané, permettent de qualifier les extraits STR-0003-P33-P00003 et STR-0003-P33-P00004 d'hormétines.

L'utilisation de ces extraits est donc adéquate dans des produits de soin pour la peau et pourront constituer une nouvelle façon de lutter contre le vieillissement cutané.

### Exemple 3 : Détermination des fractions et composés contenus dans les extraits racinaires d'Ipomoea batatas selon l'invention ayant un effet hormétique sur les fibroblastes - Etude en puces à ADN "anti-âge"

Il a été démontré dans les exemples 1 et 2 qu'un extrait *d'Ipomoea batatas* selon l'invention peut être qualifié d'hormétine.

Afin de déterminer quelle fraction de l'extrait selon l'invention est responsable de cet effet, les variations d'expression de sept marqueurs cibles (impliqués dans le vieillissement cellulaire et cutané induites par quatre fractions d'un extrait racinaire *d'Ipomoea batatas,* et par l'extrait lui-même, chez des fibroblastes ont été mesurées par qRT-PCR. Une condition correspondant à des fibroblastes NHDFs irradiés aux UVA (10J/cm²) a été analysée en parallèle comme contrôle positif du test.

Les sept marqueurs étudiés (HOMX1, NQO1, HSPA1A, GCLM, GSTP1, UGT1A6 et NOS2) sont des gènes dont l'expression est connue pour être régulée par le facteur de transcription Nrf-2, jouant un rôle majeur dans la défense antioxydante.

### 3.1. Matériels et méthodes

### Extrait

Un extrait racinaire *d'Ipomoea batatas* (PAT0014), possédant une concentration en 3,5-DCQ de 1,1g/L, a été préparé dans du DPG tel que décrit dans l'exemple 1. Le 3,5-DCQ est le composé majoritaire de cet extrait (11% en masse de l'extrait sec total). Cet extrait comprend également au moins un composé selon l'invention et dans une moindre proportion de l'acide chlorogénique et d'autres dérivés d'esters d'acide caféique et leurs isomères.

### Fractions

Les composés majoritaires de l'extrait racinaire *d'Ipomoea batatas* étudiées sont cités dans le Tableau 4 suivant :

**Tableau 4. Composés majoritaires des fractions étudiées**

| **Fractions** | **Composés majoritaires** |
|---|---|
| STL964 | 3,4-DCQ (Acide Isochlorogénique B) |
| STL965 | 3,5-DCQ (Acide Isochlorogénique A) |
| STL967 | 4,5-DCQ (Acide Isochlorogénique C) |
| STL1654 | Esters DPG de 3,5-DCQ |

Les quatre fractions comprennent également dans une moindre proportion de l'acide chlorogénique et d'autres dérivés d'esters d'acide caféique et leurs isomères. La fraction STL1654 correspond à un extrait végétal comprenant majoritairement au moins un composé de formule générale (I) selon l'invention et peut comprendre en outre des DCQ dans une moindre proportion.

Les fractions STL964, STL965, STL967 ont été isolées de l'extrait brut par purification sur colonne préparative (C18). Chaque fraction a été caractérisée par RMN (1H et 13C) et spectrométrie de masse (LC/MS).

La fraction STL1654 a été obtenue par hémisynthèse à partir de la fraction STL965, selon le procédé décrit ci-dessous :

### Réactifs

| Réactif | CAS | Fournisseurs | Formule brute | MW | Masse de l'échantillon (mg) |
|---|---|---|---|---|---|
| 3,5-DCQ - Acide Isochlorogénique A - (=STL965) | 2450-53-5 | Synthelor | C₂₅H₂₄O₁₂ | 516,45 | 100 |
| Dipropylene Glycol (DPG) / Mélange d'isomères | 25265-71-8 | | C₆H₁₄O₃ | 134,17 | 1039 |
| Amberlite IR-120H (Plus) | 78922-04-0 | Aldrich | | | 10 |

### Réaction

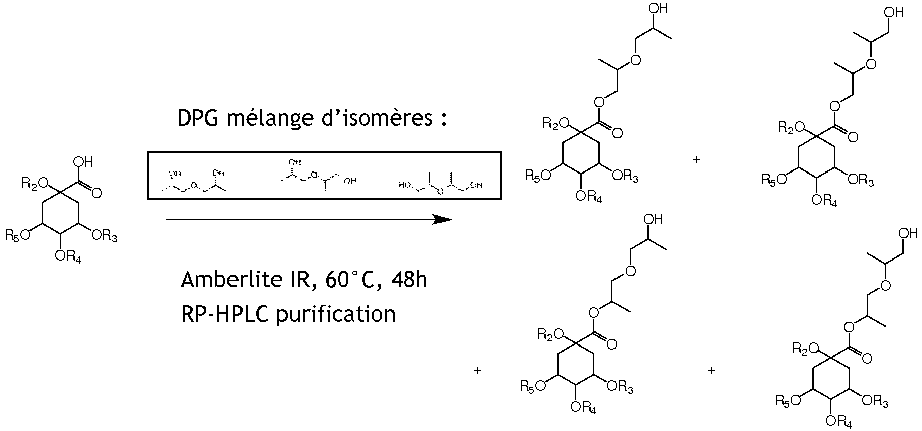

### Préparation

La fraction STL965 (100 mg, 0,194 mmol) est dissoute dans du dipropylène glycol (DPG) correspondant à un mélange d'isomères de formule (VIa), (VIb) et (VIIc) (1019 µl, 7,75 mmol). De l'Amberlite IR-120H (Plus) (10 mg, 0,194 mmol) est ajoutée et le mélange réactionnel est chauffé à 60°C pendant 72 heures sous argon et sous agitation faible dans un tube hermétiquement fermé.

Le milieu réactionnel est dilué dans du HCI 1M, filtré puis extrait 3 fois dans de l'acétate d'éthyle. Les phases organiques combinées sont successivement lavées avec du HCl 1M (1x), de la saumure, séchées sur MgSO₄, filtrées et évaporées sous vide. Le résidu obtenu est dissout dans 500µL d'une solution acétonitrile/eau 1/1.

La solution brute obtenue est ensuite purifiée par RP-HPLC sur colonne C18 (Luna, 250x21.2 mm, 5µm) à un débit de 20mL/min et par détection UV à 254nm. Les fractions pures ont été mélangées ensemble et lyophilisées pour obtenir les esters de DPG de DCQ.

### Culture Cellulaire

L'étude a été réalisée sur des fibroblastes de derme humains NHDFs (ATCC, CRL-2522, origine : prépuce) à environ 40% de leur potentiel prolifératif in vitro. Ces cellules ont été cultivées en monocouche en milieu DMEM (Invitrogen, 31885-049) contenant 10% de sérum de veau foetal (Invitrogen, 10270-106) et des antibiotiques (pénicilline/streptomycine, Invitrogen, 15140-122). Ces cellules ont été maintenues dans une atmosphère humide à 37°C contenant 5% de CO2.

### Détermination de la concentration d'analyse des 4 fractions et de l'extrait lui-même par une étude de cytotoxicité

Afin de déterminer la concentration d'analyse optimale pour les fractions, une expérience préliminaire a été réalisée sur fibroblastes de derme humains NHDFs ayant effectué 30,3 doublements de population. Cette étude a consisté à évaluer la viabilité des cellules au MTS (3-(4,5-dimethythiazol-2-yl)-5-(3-carboxy-methoxyphenyl)-2-(4-sulfophenyl)-2H-tetrazolium) (Promega, G3581) après 24h de traitement par les 4 fractions, et ce, à 5 concentrations (100 µM, 50 µM, 25 µM, 12.5 µM, et 6.25 µM) et en triples de culture (n=3).

Les fractions et l'extrait ayant été préparés dans du DMSO et du DPG70 respectivement, l'effet de ces 2 véhicules a également été testé.

Le SDS (sodium dodécyl sulfate) à 0,08% a été utilisé en tant que contrôle positif de cytotoxicité afin de valider l'expérience.

### Analyse des modifications d'expression génique

### Extraction de l'ARN total

L'extraction des ARNs totaux a été réalisée à l'aide du kit RNeasy (Qiagen, 74106), après 24h de traitement avec les fractions ou 6h après l'irradiation aux UVA. Les cellules ont été rincées avec du PBS et lysées dans du tampon ad hoc (des triples de culture ont été réalisés pour chaque condition).

L'extraction et la purification des ARNs ont été réalisées selon les instructions du fournisseur. Les ARNs totaux ont ensuite été conservés à -80°C.

### Qualification des ARNs par spectrophotométrie et électrophorèse capillaire

La concentration des ARNs totaux a été déterminée par mesure spectrophotométrique. La qualité et l'intégrité des ARNs a ensuite été vérifiée par électrophorèse capillaire (plate-forme Agilent Bioanalyzer 2100) :
- Quantification des ARN par mesure spectrophotométrique : un aliquot de chaque ARN a été dilué dans de l'eau RNAse-free et sa concentration a été déterminée à l'aide d'un spectrophotomètre Ultrospec 1100 Pro (Amersham).
- Intégrité des ARN par électrophorèse capillaire sur Bioanalyseur Agilent : l'intégrité de l'ARN total a été évaluée par la visualisation des pics d'électrophorèse correspondant aux ARNs ribosomiaux. Pour les ARN totaux d'eucaryotes supérieurs, la taille des bandes ribosomales doit être de 1,9 kb pour le 18S-ARN et de 4,7 kb pour le 28S-ARN. L'intensité de la bande supérieure doit présenter une intensité supérieure à celle de la bande inférieure. Des petites bandes diffuses représentant des ARNs de poids moléculaire plus faible (ARNt et l'ARN ribosomique 5S) peuvent être présentes. Lorsque l'ARN est dégradé, on observe un étalement des bandes de l'ARN ribosomal ainsi qu'un bruit de fond des ARN de poids moléculaire plus élevé.

### qPCR en temps réel à l'aide de sondes de type TaqMan

La méthode de qPCR en temps réel a été utilisée pour quantifier l'expression des marqueurs cibles dans les différentes populations d'ARNs. Les sondes TaqMan spécifiques ont été synthétisées par la société Applied Biosystems.

Les séquences cibles des gènes d'intérêt ont été amplifiées par PCR en utilisant les « TaqMan Gene Expression Assays » (Applied Biosystems). Ces kits spécifiques des gènes d'intérêt comprennent une sonde TaqMan et 2 amorces spécifiques, qui ont été pré-mélangées à une concentration de 18µM pour chaque amorce et de 5µM pour la sonde. Ce mélange est 20 fois concentré. Les sondes TaqMan ont été greffées avec un fluorophore (FAM) à l'extrémité 5' de la séquence et avec un quencher de fluorescence en 3'.

Les PCR ont été réalisées à l'aide du système 7900HT Fast Real-Time PCR (Applied Biosystems). Les réactions ont été réalisées dans un volume de 20µl.

Le mélange réactionnel contient 10µl de TaqMan Fast Universal Master Mix (Applied Biosystems), 1µl de TaqMan Gene Expression Assay et 5µl d'eau RNAse-free. Dans chaque puits d'une microplaque 96-puits, 16µl du mélange et 4µl d'ADNc (4ng) ont été ajoutés. A des fins de normalisation, des mélanges réactionnels avec des sondes et amorces correspondant à la glycéraldéhyde-3-phosphate déhydrogenase (GAPDH) ont également été préparés avec les mêmes échantillons d'ADNc. Un contrôle sans ADNc a servi de contrôle négatif d'amplification. Les cycles thermiques ont été programmés avec une étape d'incubation à 50 °C pendant 2 minutes, suivie d'une première étape de dénaturation à 95 °C pendant 10 minutes. Le protocole d'amplification PCR s'est poursuivi avec 40 cycles de 15 secondes à 95 °C et 1 minute à 60 °C.

Les niveaux relatifs d'expression ont été quantifiés selon la méthode de calcul de l'expression relative par rapport à un gène de ménage (2-Ct), dérivée du calcul des ΔΔCt (Pfaffl, 2001 & Livak and Schmittgen, 2001).

### 3.2. Résultats

### Détermination de la concentration d'analyse des 4 fractions par une étude de cytotoxicité

Afin de déterminer la concentration optimale d'étude des 4 fractions, une étude de cytotoxicité a été réalisée sur les fibroblastes de derme humains NHDFs, sur la base de 5 concentrations après 24h de contact (n=3). Les paramètres expérimentaux étaient identiques à ceux utilisés par la suite pour l'étude des effets sur l'expression génique en termes de passages en culture des cellules, de confluence et de temps de contact. L'effet des 2 véhicules (DPG70 et DMSO) a été testé en parallèle.

Les résultats ont été comparés au contrôle non traité et le SDS à 0,08% a été utilisé comme contrôle positif de cytotoxicité afin de valider l'expérience. Le seuil de cytotoxicité a été arbitrairement fixé à 80% de viabilité.

Les résultats sont présentés à la **Figure 6** et montrent que les fractions STL964, STL965, STL967 et STL1654 ainsi que le véhicule DMSO à 1% ne présentent pas de cytotoxicité.

Sur base de ces résultats et afin de comparer les effets des fractions, la concentration optimale de travail pour chacun d'eux a été fixée à 50 µM.

### Qualification des ARNs par électrophorèse capillaire

La qualité et l'intégrité des ARNs extraits ont été évaluées par électrophorèse capillaire. Les profils obtenus témoignent de l'intégrité des différentes populations d'ARN. Les échantillons ont dès lors pu être utilisés dans les réactions de synthèse des ADNs complémentaires.

### Analyse des modifications d'expression de gènes induites par les différentes fractions d'extrait végétal

Parmi les cinq candidats étudiés, aucune expression n'a été détectée pour UGT1A6 et NOS2, quelle que soit la condition étudiée. Les gènes correspondants ne semblent donc pas être exprimés par les fibroblastes NHDFs. Les profils d'expression des ARNm ciblant HMOX1, HSPA1A, NQO1, GCLM et GSTP1 après traitement des fibroblastes NHDFs avec les fractions et l'extrait sont illustrés dans les **Figures 7 à 11****.** Les niveaux d'expression sont exprimés en pourcentages par rapport aux contrôles respectifs.

La condition contrôle irradiée aux UVA (10 J/cm2) a été comparée à la condition contrôle PBS, les conditions traitées par les fractions STL964, STL965, STL967 et STL1654 ont été comparées à la condition contrôle DMSO 0,5% (véhicules de ces fractions) et les conditions traitées par l'extrait *d'Ipomoea batatas* PAT0014 ont été comparées à la condition contrôle DPG70 2,5% (véhicule de l'extrait).

L'analyse statistique effectuée est basée sur un test t-student comparant individuellement chaque traitement à son contrôle (* : p < 0.05 ; ** : 0,001< p < 0.01 et *** : p < 0,001). Les valeurs p < 0,05 ont été considérées comme significatives, les valeurs 0,001 < p < 0,01 comme hautement significatives et les valeurs p < 0,001 comme très hautement significatives.

Les **Figures 9 et 10** montrent que, parmi les fractions testées au cours de la présente étude, la fraction STL1654 contenant les esters de DPG de DCQ permet d'induire de façon très significative l'expression du gène NQO1 codant pour la NAD(P)H déshydrogénase quinone 1 **(****Figure 9****)** et du gène GCLM codant pour la glutamate cystéine ligase **(****Figure 10****).**

La fraction STL1654 contenant les esters de DPG de DCQ présente également une tendance à induire une surexpression du gène HMOX-1 codant pour l'hème oxygénase-1 (HO-1) **(****Figure 7****),** du gène HSPA1A codant pour la protéine HSP70 **(****Figure 8****),** et du gène GSTP1 codant pour la glutathion S transférase pi **(****Figure 11****).**

Enfin, les Figures 2 à 6 montrent que l'extrait PAT0014 permet d'induire de façon très significative une surexpression du gène HMOX-1 **(****Figure 7****)** et du gène GCLM **(****Figure 10****),** mais n'a aucune influence sur l'expression des gènes HSPA1A **(****Figure 8****),** NQO1 **(****Figure 9****)** et GSTP1 **(****Figure 11****).**

Contrairement à la fraction STL1654, les fractions STL964, STL965 et STL967 contenant des DCQ n'induisent pas de surexpression des gènes d'intérêt **(****Figures 7 à 11****).**

### 3.3. Conclusions

L'induction du gène de réponse au stress Nrf-2, démontre qu'un extrait comprenant au moins un composé de formule générale (I) selon l'invention, exerce un effet protecteur vis-à-vis de stress radicalaires endogènes liés au vieillissement chronologique, ou exogènes tels qu'induits par les UVA ou divers agents polluants, et ce par un mécanisme basé sur le principe d'hormesis.

L'étude des différentes fractions d'un extrait *d'Ipomoea batatas* permet également de démontrer que les esters de DPG de DCQ sont à l'origine de cet effet protecteur vis-à-vis du stress oxydatif, contrairement aux DCQ.

### Exemple 4 : Préparation et effet d'extraits racinaires d'Ipomoea batatas dans du DPG ou du propane 1,3 diol, sur des fibroblastes humains et dans des épidermes humains reconstitués mélanisés - Etude en puces à ADN "GeneChip Human Gene"

Afin d'analyser la possibilité qu'un extrait selon l'invention possède une action anti-âge, une étude transcriptomique sur puce à ADN a été réalisée à partir de fibroblastes humains et d'épidermes humains reconstitués mélanisés traités durant 24 heures par deux extraits racinaires *d'Ipomoea batatas* comprenant au moins soit un ester de DPG de DCQ soit un ester de propane 1,3 diol de DCQ selon l'invention. Les variations d'expression des gènes ont été contextualisées et interprétées biologiquement au travers de la base de données « StratiCELL Skin Knowledge database ».

### 4.1. Matériels et méthodes

### Extraits

Un extrait racinaire *d'Ipomoea batatas,* possédant respectivement une concentration en 3,5-DCQ de 1,1 ± 0,1g/L a été préparé par exsudation racinaire dans du DPG de plante *d'Ipomoea batatas* cultivées hors sol (ci-après extrait n°2). Le 3,5-DCQ est le composé majoritaire de cet extrait (12-15% en masse de l'extrait sec total). Cet extrait comprend également dans une moindre proportion de l'acide chlorogénique, des esters de DPG de DCQ et d'autres dérivés d'esters d'acide caféique et leurs isomères. Il s'agit donc d'un mélange de composés de formule (I), dans lequel le 3,5-DCQ est présent de façon très majoritaire. Le teneur en solvant (DPG) en pourcentage v/v est de 70 ± 5 %, le pH de l'extrait est 3,0 ± 0,1. Dans l'exemple 4, cet extrait est intitulé extrait n°2.

Un extrait racinaire *d'Ipomoea batatas,* possédant une concentration en ester de propane 1,3 diol de DCQ de 1,3 ± 0,3 g/L dont 1,0 ± 0,1 g/L d'ester de propane 1,3 diol de 3,5-DCQ, a été préparé par exsudation racinaire dans du propane 1,3 diol biosourcé de plante *d'Ipomoea batatas* cultivées hors sol (ci-après extrait n°1). La conversion du 3,5-DCQ en ester de propane 1,3 diol de 3,5-DCQ est supérieure à 95%. Ainsi, cet extrait comprend également dans une moindre proportion de l'acide chlorogénique, des acides dicaféoylquiniques et leurs isomères. Il s'agit donc d'un mélange de composés de formule (I), dans lequel l'ester de propane 1,3 diol de 3,5-DCQ est présent de façon très majoritaire. La teneur en solvant est ajustée avec de l'eau jusqu'à un pourcentage v/v de 55 ± 5 %, le pH de l'extrait est de 4,2 ± 0,2. Dans l'exemple 4, cet extrait est intitulé extrait n°1.

### Culture Cellulaire

La première partie de l'étude a été réalisée sur des fibroblastes de derme humains NHDFs (ATCC, CRL-2522, origine : prépuce) à environ 40% de leur potentiel prolifératif in vitro. Ces cellules ont été cultivées en monocouche en milieu DMEM (Invitrogen, 31885-049) contenant des antibiotiques (pénicilline/streptomycine, Invitrogen, 15140-122). Ces cellules ont été maintenues dans une atmosphère humide à 37°C contenant 5% de CO2.

La seconde partie de l'étude a été réalisée sur des épidermes reconstitués (StratiCELL®, RHE/MEL/001) contenant ou non des mélanocytes humains primaires NHEMs (Normal Human Epidermal Melanocytes) provenant d'un donneur de phototype foncé (phototype IV à V) (Invitrogen, C2025C, lot n°439684). Les tissus ont été cultivés à l'interface air-liquide durant 14 jours dans un milieu de culture approprié, et une atmosphère humide à 37°C contenant 5% de CO2.

### Détermination de la gamme de concentrations d'étude des deux extraits racinaires d'Ipomoea batatas par une étude préliminaire de cytotoxicité

Afin de déterminer la concentration d'analyse optimale pour les deux extraits, une expérience préliminaire a été réalisée sur fibroblastes NHDFs, sur mélanocytes humains NHEMs et sur épidermes reconstitués.

Les fibroblastes NHDFs ont été ensemencés en plaques 24 puits 24 heures avant l'application des extraits. Les mélanocytes humains primaires NHEMs ont été ensemencés en plaques 24 puits 24 heures avant l'application des extraits. Les épidermes reconstitués (RHE/001 ; lot CB0314/2) ont été transférés sur une plaque 12 puits avant d'être traités avec les extraits.

Les deux extraits ont été solubilisés dans le milieu de culture, puis placés en contact des fibroblastes humains NHDFs, des mélanocytes humains primaires NHEMs, ou des épidermes différenciés durant 24 heures.

Cette étude a consisté à évaluer la viabilité des cellules et des épidermes au MTS (3-(4,5-dimethythiazol-2-yl)-5-(3-carboxy-methoxyphenyl)-2-(4-sulfophenyl)-2H-tetrazolium) (Promega,G3581), 24 heures après l'ajout des deux extrait végétaux et les deux solvants (DPG 70% pH = 3 et propane 1,3 diol 56% pH = 4,2) et ce, à 5 concentrations et 3 répétions (n=3) pour les fibroblastes NHDFs et les mélanocytes NHEMs, et à 2 concentrations et 3 répétitions (n=3) pour les épidermes reconstitués.

Le SDS (sodium dodécyl sulfate) est toxique pour les cellules et a été utilisé en tant que contrôle positif afin de valider l'expérience.

Au terme de cette expérience, une concentration non cytotoxique a été définie afin de procéder à l'analyse transcriptomique.

Les concentrations suivantes, en pourcentage v/v, ont été testées :
- Fibroblastes NHDFs et les mélanocytes NHEMs: 3%, 1%, 0,3% 0,1%, 0,03%
- Epidermes reconstitués : 1% et 0,3%

### Traitement des cellules

Les extraits et solvants ont été appliqués à une concentration choisie, pendant 24h, dans le milieu de culture des fibroblastes NHDFs et des épidermes pigmentés. Des quadruples de culture ont été réalisés pour chaque condition (n=4). Au terme des traitements, les populations d'ARNs totaux ont été extraites. Les ARNs ont été quantifiés par spectrophotométrie (n=4) et leur intégrité a été analysée par électrophorèse capillaire (pour des triples de chaque conditions ; n=3).

### Extraction de l'ARN total

L'extraction des ARNs totaux a été réalisée à l'aide du kit RNeasy (Qiagen).

Après 24h de traitement, les cellules ont été rincées avec du PBS et lysées dans du tampon ad hoc, alors que les épidermes ont été découpés de l'insert et directement plongés dans ce tampon.

L'extraction et la purification des ARNs ont été réalisées selon les instructions du fournisseur. Les ARNs totaux ont ensuite été conservés à -80°C en vue de l'analyse transcriptomique.

### Qualification des ARNs par spectrophotométrie et électrophorèse capillaire

La concentration des ARNs totaux a été déterminée par mesure spectrophotométrique. La qualité et l'intégrité des ARNs ont ensuite été vérifiées par électrophorèse capillaire (plate-forme Agilent Bioanalyzer 2100 - Agilent RNA 6000Nano Kit, 5067-1511).
- Quantification des ARN par mesure spectrophotométrique : un aliquot de chaque ARN a été dilué dans de l'eau RNAse-free et sa concentration a été déterminée à l'aide d'un spectrophotomètre Ultrospec 1100 Pro (Amersham).
- Intégrité des ARN par électrophorèse capillaire sur Bioanalyseur Agilent : l'intégrité de l'ARN total a été évaluée par la visualisation des pics correspondant aux ARNs ribosomiaux. Pour les ARN totaux d'eucaryotes supérieurs, la taille des bandes ribosomales doit être de 1,9 kb pour le 18S-ARN et de 4,7 kb pour le 28S-ARN. L'intensité de la bande correspondant au 28S-ARN doit être supérieure à l'intensité de la bande correspondant au 18S-ARN (profil de gauche ci-après). Des petites bandes diffuses représentant des ARNs de poids moléculaire plus faible (ARNt et l'ARN ribosomique 5S) peuvent être présentes. Lorsque l'ARN est dégradé, on observe un étalement des bandes de l'ARN ribosomal ainsi qu'un bruit de fond des ARNs de poids moléculaire plus élevé.

### Phase d'hybridation sur puces GeneChip Human Gene 2.0 ST (Affymetrix)

Les ARNs ont ensuite été dilués à 50 ng/µl et 50 µl de chaque échantillon (n=3). La quatrième réplique sert de back-up.

Les échantillons d'ARN (50ng) ont été amplifiés par l'utilisation de la technologie Ribo-SPIA, selon 3 étapes (Ovation Pico WTA System V2, NuGEN, 3302-12) et purifiés avec le kit Agencourt RNA Clean up XP Beads (Agencourt - Beckam Coulter Genomics, A29168). Pour chaque échantillon, 4,5 µg ont été fragmentés et marqués à la biotine, grâce au NuGEN Encore Biotin Module (NuGEN, 4200-12). L'hybridation a été effectuée sur des puces à ADN du modèle GeneChip Human Gene 2.0 ST (Affymetrix, 902112). Les étapes d'hybridation sur puce, lavage et fixation, ont été réalisées suivant le protocole défini par Affymetrix. La solution d'hybridation a été préparée par utilisation des kits Affymetrix Genechip Expression 3' Amplification Reagent Hybridization Controls (Affymetrix, 900454) et Hybridization Module for GeneChip Hybridization, Wash and Stain Kit (Affymetrix,900720), et mélangée avec l'ADN complémentaire (ADNc) amplifié dans les étapes précédentes.

L'hybridation a été réalisée durant 18h dans le four GeneChip Hybridization Oven 640 (Affymetrix,800139). Le lavage et la révélation ont été réalisés à l'aide de la station fluidique GeneChip Fluidics Station 450 (Affymetrix, 00-0079) et les mesures d'intensité ont été scannées avec le GeneChip Scanner 3000 (Affymetrix).

### Prétraitement des données d'hybridation

Le traitement des données brutes a été réalisé avec les logiciels R (v3.2.3 ; Ihaka and Gentleman, 1996) et le package 'oligo' (v1.34.2 ; Carvalho BS and Irizarry AR, 2010) du projet BioConductor (v3.2 ; Gentleman et al, 2004). La dernière version des librairies fournies par Affymetrix, construites sur la version 19 du génome humain (UCSC Human genome 19), et la méthode RMA, décrite par Irizarri et al. (Irizarri et al, 2003a ; Irizarri et al, 2003b) ont été utilisées afin de guider et effectuer le prétraitement et l'annotation des séquences.

### Analyse des données, annotation et analyse thématique

L'analyse statistique individuelle des gènes/transcrits a été réalisée avec les méthodes "Moderated t" et "Moderated F" implémentées dans le package R Limma 3.26.8 (Smyth GK, 2004).

L'annotation des gènes et la définition de groupes de gènes pour l'analyse de sur-représentation ont été réalisés au départ de la base de données interne « StratiCELL Skin Knowledge Database » (Salmon M & Berger F, 2014).

L'analyse de sur-représentation a été réalisée avec la méthode du test hypergéométrique dans but de caractériser les facteurs thèmes/groupes d'intérêt dermo-cosmétique au départ de la proportion de leurs cibles détectées et différentiellement exprimées. L'analyse a été menée au départ de la liste des gènes détectés avec une p-value de 0.05 et une différence du niveau d'expression (Taux de variation ou fold-change ou FC) supérieur à 1,5 (bi-latéral).

### 4.2. Résultats

### Détermination de la concentration d'analyse des deux extraits racinaires d'Ipomoea batatas par une étude préliminaire de cytotoxicité

Une étude de la cytotoxicité a été réalisée sur des fibroblastes NHDFs et sur des épidermes reconstruits ainsi que sur des mélanocytes primaires humains NHEMs afin d'exclure une éventuelle cytotoxicité spécifique sur ces cellules, présentes en faible nombre au sein des épidermes reconstruits. Les extraits et les solvants respectifs utilisés pour la préparation de ceux-ci, ont été appliqués dans les milieux de culture, durant 24h (n=3). Le contrôle non traité a été arbitrairement fixé à 100% de viabilité et le seuil de cytotoxicité a été fixé par convention à 80% de viabilité. La condition SDS constitue le contrôle positif qui valide l'expérience.

Sur la base des résultats de l'étude préliminaire de cytotoxicité (données non montrées), les concentrations optimales (en pourcentage v/v) sélectionnées pour chacun des deux extraits et les solvants sont les suivantes :
- Extrait 1 et propane 1,3 diol : pour les fibroblastes NHDFs 3% et pour les mélanocytes NHEMs ainsi que les épidermes reconstitués 0,3%.
- Extrait 2 et DPG: pour les fibroblastes NHDFs 3% et pour les mélanocytes NHEMs ainsi que les épidermes reconstitués 0,3%.

### Quantification des ARNs par spectrophotométrie

Le rapport de l'absorbance à 260nm et 280nm est utilisé pour évaluer la pureté de l'ARN. Un rapport proche de 2 permet de considérer l'échantillon comme étant pur et dépourvu de contamination par des protéines. Le rapport 260/230 est utilisé en tant que mesure secondaire de la pureté de l'échantillon. La valeur 260/230 est généralement plus élevée que le ratio 260/280 et est attendue aux environs de 2,2. Les ratios obtenus pour l'ensemble des échantillons de la présente étude sont donc satisfaisants (données non montrées) et ont été qualifiés ensuite par électrophorèse capillaire.

### Qualification des ARNs par électrophorèse capillaire

Les différentes populations d'ARN démontrent bien la présence de pics étroits, correspondant aux ARN ribosomiaux 18S et 28S, et un rapport équilibré entre les deux pics. L'absence de pics intermédiaires et étalés, caractéristiques de produits de dégradation des ARNs témoigne de l'intégrité des différentes populations (données non montrées).

La qualité et l'intégrité des ARNs extraits étant démontrées, ceux-ci ont dès lors été utilisés pour la poursuite du protocole et engagés dans les réactions d'amplification, de purification, marquage à la biotine puis hybridation sur puces à ADN.

### Analyse des modifications d'expression de gènes induites par l'extrait n°2

### 1) Effets de l'extrait n°2 sur le transcriptome des fibroblastes de derme humains NHDFs.

L'extrait n°2 a été ajouté dans le milieu de culture des fibroblastes NHDFs (n=4) à une concentration v/v de 3%. Un contrôle traité seulement par le solvant DPG 70% (véhicule de l'extrait, concentration v/v de 3%, n=4) a également été analysé. Après 24h de culture des fibroblastes NHDFs, les différences d'expression géniques ont été analysées par hybridation sur puces à ADN.

Quelques résultats significatifs sont présentés de manière récapitulative sous la forme d'un tableau (Tableau 5) indiquant des gènes représentatifs d'un effet cosmétique bénéfique, variant significativement, 24h après l'application de l'extrait n°2 sur des fibroblastes de derme humains NHDFs.

**Tableau 5 : Exemples de gènes qui varient de manière significative 24 h après l'application de l'extrait racinaire n°2 (à 3%) sur des fibroblastes de derme humains NHDFs. Le symbole des gènes, le nom des gènes, la variation de l'expression (FC) par rapport au véhicule DPG 70% (à 3%) (FC > 1 : augmentation - FC < 1 : diminution) et la valeur p (p-value) sont présentés.**

| p-value | Taux de variation (FC) | Symbole | Nom |
|---|---|---|---|
| 1.0e-6 | 2,8 | HMOX1 | hème oxygénase -1 |
| 6.4e-5 | 3,02 | MT1G / MT1K | métallothionéine 1G / métallothionéine 1K |
| 5.2e-4 | 1,45 | FTH1 | ferritine, |
| 1.5e-2 | 1,22 | NQO1 | NAD(P)H déshydrogénase quinone 1 |
| 2.3e-2 | 1,69 | MT1H | métallothionéine 1H |

L'extrait n°2 induit la surexpression des métallothionéines 1G (MT1G) et 1H (MT1H) connues pour leur activité détoxifiante et anti-radicalaire. Les métallothionéines (MTs) sont des protéines de faible poids moléculaire (6-10 kDa), contenant de nombreux résidus cystéines, et deux domaines de chélation des métaux lourds. Elles peuvent être activées par différents stimuli, tels que les métaux lourds, les UV, des cytokines inflammatoires ou encore certains facteurs de croissance. Les MTs jouent un rôle crucial dans le maintien de l'homéostasie des métaux physiologiques et la détoxification des métaux toxiques issu de la pollution urbaine, tels que le cuivre, le nickel, le mercure ou le cadmium. Elles sont également largement impliquées dans la protection contre le stress oxydant, en particulier au niveau de la mitochondrie où leur présence est finement régulée par le niveau de stress lié à la respiration.

En cohérence avec les exemples précédents 1 à 3, l'extrait n°2 induit une augmentation d'expression des gènes HMOX et NQO1. Le gène HMOX1 code pour l'hème oxygénase (HO-1) qui est impliquée dans la dégradation de l'hème et est bien connue pour son rôle protecteur contre le stress oxydant.

Son rôle protecteur est essentiellement dû à sa capacité à dégrader l'hème, ayant des propriétés prooxydantes en ions Fe2+, monoxide de carbone et en biliverdine, précurseur d'un puissant métabolite antioxydant, la bilirubine. La NAD(P)H déshydrogénase, quinone 1 (NQO1) est une flavoprotéine cytosolique sous le contrôle du facteur de transcription NRF-2. NQO1 favorise, par réduction la formation des hydroquinones à partir des quinones, empêchant la production d'espèces radicalaires. NQO1 est surexprimée après contact avec des agents pro-oxydants et des métaux lourds, après exposition aux UV ou à des radiations ionisantes, afin de protéger la cellule de leurs effets néfastes.

Aussi, nous observons une stimulation du gène codant pour la chaîne lourde de la ferritine (FTH1). Celle-ci est souvent surexprimée suite à une exposition à un stress radicalaire. Elle est responsable du stockage du fer, issu notamment de la dégradation de l'hème, dans une forme non nocive et participe à son transport et à la régulation de sa disponibilité en fonction des circonstances. En effet, le fer catalyse de nombreuses réactions bénéfiques mais aussi des réactions nocives comme la peroxydation des lipides et la réaction de Fenton produisant des radicaux hydroxyles.

### 2) Effets de l'extrait n°2 sur le transcriptome d'épidermes humains reconstitués mélanisés.

L'extrait n°2 a été ajouté dans le milieu de culture des épidermes mélanisés (n=4) à une concentration v/v de 0,3%. Un contrôle traité seulement par le solvant DPG 70% (véhicule de l'extrait, concentration v/v de 0,3%, n=4) a également été analysé. Après 24 h de culture des épidermes mélanisés, les différences d'expression géniques ont été analysées par hybridation sur puces à ADN.

Quelques résultats significatifs sont présentés de manière récapitulative sous la forme d'un tableau (Tableau 6) indiquant des gènes représentatifs d'un effet cosmétique bénéfique, variant significativement, 24h après l'application de l'extrait n°2 sur des épidermes humains mélanisés.

**Tableau 6 : Exemples de gènes qui varient de manière significative 24 h après l'application de l'extrait racinaire n°2 (à 0,3%) sur des épidermes humains mélanisés. Le symbole des gènes, le nom des gènes, la variation de l'expression (FC) par rapport au véhicule DPG 70% (à 0,3%) (FC > 1 : augmentation - FC < 1 : diminution) et la valeur p (p-value) sont présentés.**

| p-value | Taux de variation (FC) | Symbole | Nom |
|---|---|---|---|
| 2,9e-8 | -6,3 | SDC2 | syndecan protéoglycan 2 |
| 1,3e-6 | -8,74 | FGF2 | facteur de croissance 2 des fibroblastes |
| 2,8e-6 | -4,84 | FGF7 / KGF | facteur de croissance 7 des fibroblastes |
| 3,5e-4 | 1,72 | CALML3 | calmoduline-like protéine 3 |
| 5,5e-3 | -1,62 | PAX3 | facteur de transcription paired box 3 |
| 2,0e-3 | 1,69 | PDE7A | phosphodiestérase 7A |
| 2,9e-5 | 2,22 | LAMC2 | laminine gamma 2 |
| 1,3e-4 | 1,71 | LAMA3 | laminine alpha 3 |
| 1,6e-3 | 1,6 | LAMB3 | laminine beta 3 |

L'extrait n°2 induit plusieurs variations d'expression de gènes clés impliqués dans la pigmentation cutanée et stimule l'expression des trois sous-unités α3, β3 et γ2 de la laminine 5, composant majeur de la jonction dermo-épidermique.

### • Pigmentation cutanée :

Les gènes codant pour la phosphodiestérase 7A (PDE7A) et pour la calmoduline-like protéine-3 (CALML3) impliqués dans la régulation de la concentration intracellulaire d'adénosine monophosphate cyclique (3'5'AMPc), second messager essentiel produit suite à l'activation par la α-MSH de l'adénylate cyclase et requis pour stimuler la protéine kinase A (PKA) en aval de la voie, menant à l'expression du facteur de transcription MITF, à l'expression de la tyrosinase et à la synthèse de mélanine, sont modulés positivement.

La PDE7A fait partie de la famille des phosphodiestérases régulant la production intracellulaire d'AMP cyclique au travers de son hydrolyse en sa forme 5'AMP inactive, régulant de la sorte négativement la voie de signalisation α-MSH/MC1R.

En outre, les phosphodiestérases peuvent être actives par les complexes Ca2+/calmoduline (CaM). Le processus débute par la liaison d'ions Ca2+ à la calmoduline ou aux protéines calmoduline-like (CALM) générant une conformation active. La forme active s'associe ensuite aux phoshodiestérases avec activation de l'enzyme.

La surexpression de la calmoduline-like protéine-3 pourrait donc renforcer l'activité des phosphodiestérases, limiter la voie α-MSH/MC1R et la synthèse de mélanine.

Le gène codant pour le facteur de croissance KGF/FGF-7 est largement sous-exprimé. Il est bien démontré que le KGF/FGF-7 favorise le transfert des mélanosomes au travers de l'activation de son récepteur spécifique présent à la surface des kératinocytes voisins (FGFR2b/KGFR), stimulant de la sorte le processus de phagocytose des mélanosomes. En outre, le facteur de croissance KGF/FGF-7 est bien décrit comme facteur d'initiation de lésions hyper-pigmentaires comme le lentigo solaire ou le mélasma.

En limitant fortement la présence de KGF/FGF-7, l'extrait n°2 constitue un excellent candidat pour le traitement et/ou la prévention de ce type de lésions caractérisées par une hyperpigmentation localisée (tâches pigmentaires).

L'extrait n°2 réprime fortement l'expression du gène codant pour le protéoglycan Syndecan-2. L'extinction du gène SDC2 par un siRNA spécifique est associée à une réduction de la synthèse de mélanine, tandis que sa surexpression induit l'effet inverse. De plus, l'expression de SDC2 est augmentée suite à une exposition aux UVB, et cette augmentation est requise pour observer une induction de la synthèse de mélanine dans ces conditions.

Enfin, nous observons une diminution du gène codant pour le facteur de transcription Paired Box 3 (PAX3), ainsi qu'une diminution du gène codant pour le facteur de croissance des fibroblastes (FGF2), bien documenté pour son rôle de régulateur de la viabilité des mélanocytes au travers de l'expression STAT3-dépendante de PAX3. Une diminution d'expression du gène FGF2 est donc logiquement liée à une répression du gène PAX3.

L'ensemble de ces données souligne un possible effet modulateur de l'extrait n°2 de la pigmentation de l'épiderme.

### • Elasticité et résistance mécanique de la peau :

La jonction dermo-épidermique (ou JDE) supporte mécaniquement l'épiderme, maintient le contact et les échanges entre ce dernier et le derme sous-jacent, et assure une fonction de barrière et de filtre sélectif. Au cours du vieillissement cutané, on observe un aplanissement et un amincissement de la JDE. Cet aplatissement va élargir les sillons du réseau microdépressionnaire de surface (réseau de sillons plus ou moins profonds qui creusent la surface de l'épiderme), et des ridules vont faire leur apparition. La jonction dermo-épidermique constitue dès lors une cible majeure en vue d'une stratégie anti-âge.

Nous observons de façon tout à fait remarquable l'induction conjointe des trois sous-unités de la laminine 5, à savoir la laminine α3 (LAMA3), la laminine β3 (LAMB3) ainsi que la laminine γ2 (LAMC2). La laminine 5 ou laminine 332, constituée de trois sous-unités α3, β3 et γ2, joue un rôle essentiel au sein de la JDE. Via son interaction avec des intégrines, elle assure l'ancrage des kératinocytes de l'épiderme aux fibres du derme, afin de former un réseau complexe qui joue un rôle de support mécanique.

### Analyse des modifications d'expression de gènes induites par l'extrait n°1

### 1) Effets de l'extrait n°1 sur le transcriptome des fibroblastes de derme humains NHDFs.

L'extrait n°1 a été ajouté dans le milieu de culture des fibroblastes NHDFs (n=4) à une concentration v/v de 3%. Un contrôle traité seulement par le solvant propane 1,3 diol 56% (véhicule de l'extrait, concentration v/v de 3%, n=4) a également été analysé. Après 24 h de culture des fibroblastes NHDFs, les différences d'expression géniques ont été analysées par hybridation sur puces à ADN.

Quelques résultats significatifs sont présentés de manière récapitulative sous la forme d'un tableau (Tableau 7) indiquant des gènes représentatifs d'un effet cosmétique bénéfique, variant significativement, 24h après l'application de l'extrait sur des fibroblastes de derme humains NHDFs.

**Tableau 7 : Exemples de gènes qui varient de manière significative 24 h après l'application de l'extrait racinaire n°1 (à 3%) sur des fibroblastes de derme humains NHDFs. Le symbole des gènes, le nom des gènes, la variation de l'expression (FC) par rapport au véhicule propane 1,3 diol (à 3%) % (FC > 1 : augmentation - FC < 1 : diminution) et la valeur p (p-value) sont présentés.**

| p-value | Taux de variation (FC) | Symbole | Nom |
|---|---|---|---|
| 4,7e-4 | -1,59 | FGF7 / KGF | facteur de croissance 7 des fibroblastes / facteur de croissance kératinocyte |
| 3,9e-3 | -1,33 | KITLG / SCF | KIT ligand ou facteur de cellules souches |
| 1,0e-2 | -1,44 | HGF | facteur de croissance hépatocyte |

### • Pigmentation cutanée :

Il est maintenant bien démontré que les signaux émis par les fibroblastes du derme influencent fortement la pigmentation cutanée.

De plus, dans certaines pathologies cutanées se manifestant par une hyperpigmentation locale, comme la sclérodermie, les macules 'café-au-lait' de la neurofibromatose ou encore le lentigo solaire, une augmentation de facteurs de croissance pro-mélanogéniques a été observée au sein du compartiment dermique. Ces facteurs ont été identifiés comme le « stem cell factor » (facteur de cellules souches, SCF ou Kit ligand), l'« hepatocyte growth factor » (facteur de croissance des hépatocytes, HGF) et le « keratinocyte growth factor » (facteur de croissance des kératinocytes, KGF/FGF-7). En outre, comme nous l'avons évoqué précédemment pour l'extrait n°2, le KGF/FGF-7 favorise le transfert des mélanosomes au travers de l'activation de son récepteur présent à la surface des kératinocytes, stimulant le processus de phagocytose des mélanosomes et la pigmentation du tissu. De façon remarquable, l'extrait n°1 inhibe l'expression de ces trois facteurs simultanément (Tableau 7).

### 2) Effets de l'extrait n°1 sur le transcriptome d'épidermes humains reconstitués mélanisés.

L'extrait n°1 a été ajouté dans le milieu de culture des épidermes mélanisés (n=4) à une concentration v/v de 0,3%. Un contrôle traité seulement par le solvant propane 1,3 diol 56% (véhicule de l'extrait, concentration v/v de 0,3%, n=4) a également été analysé. Après 24 h de culture des épidermes mélanisés, les différences d'expression géniques ont été analysées par hybridation sur puces à ADN.

Quelques résultats significatifs sont présentés de manière récapitulative sous la forme d'un tableau (Tableau 8) indiquant des gènes représentatifs d'un effet cosmétique bénéfique, variant significativement, 24h après l'application de l'extrait sur des épidermes humains mélanisés.

**Tableau 8 : Exemples de gènes qui varient de manière significative 24 h après l'application de l'extrait racinaire n°1 (à 0,3%) sur des épidermes humains mélanisés. Le symbole des gènes, le nom des gènes, la variation de l'expression (FC) par rapport au véhicule, le solvant propane 1,3 diol 56% (à 0,3%) (FC > 1 : augmentation - FC < 1 : diminution) et la valeur p (p-value) sont présentés.**

| p-value | Taux de variation (FC) | Symbole | Nom |
|---|---|---|---|
| 3,0e-2 | 1,28 | CALML3 | calmoduline-like protéine 3 |
| 5,3e-4 | 1,5 | PDE7A | phosphodiestérase 7A |
| 8,2e-6 | -2.22 | KIF20A | famille des kinésines membre 20A |
| 1,5e-4 | -1,73 | KIF15 | famille des kinésines membre 15 |
| 1,3e-3 | -1,82 | KIF11 | famille des kinésines membre 11 |
| 4,3e-3 | -1,57 | KIF24 | famille des kinésines membre 24 |
| 4,6e-3 | -1,59 | KIF18A | famille des kinésines membre 18A |
| 1,1e-2 | -1,52 | KIF14 | famille des kinésines membre 14 |
| 1,2e-2 | -1,58 | KIF23 | famille des kinésines membre 23 |
| 1,5e-5 | 2,28 | LAMC2 | laminine gamma 2 |
| 1,4e-4 | 1,62 | LAMA3 | laminine alpha 3 |
| 1,2e-3 | 1,61 | LAMB3 | laminine beta 3 |

L'extrait n°1 induit plusieurs variations d'expression de gènes clés impliqués dans la pigmentation cutanée et stimule l'expression des trois sous-unités α3, β3 et γ2 de la laminine 5, composant majeur de la jonction dermo-épidermique.

### • Pigmentation cutanée :

Tout comme pour l'extrait n°2, nous observons une surexpression de deux gènes, à savoir la phosphodiestérase 7B (PDE7A) et la calmoduline-like protéine-3 (CALML3), impliqués dans l'activité AMPc phosphodiestérase (PDE), modulant la concentration intracellulaire d'adénosine monophosphate cyclique (3'5'AMPc), second messager produit suite à l'activation par l'α-MSH de l'adénylate cyclase. Une diminution de la concentration intracellulaire d'AMPc pourrait résulter en une réduction de synthèse de mélanine.

En parallèle, nous observons une sous-expression de plusieurs membres de la famille des kinésines (gènes KIF), protéines motrices impliquées dans le transport des mélanosomes vers l'extrémité des dendrites des mélanocytes.

Les mélanosomes constituent des organites intracellulaires spécifiques des mélanocytes, au sein desquels la mélanine est synthétisée et stockée. Il est maintenant bien établi que l'inhibition du transfert des mélanosomes constitue une stratégie pour la conception d'un actif dépigmentant.

Il a été démontré que les kinésines sont inactivées lorsque la concentration d'AMPc chute, entrainant une agrégation des mélanosomes dans les cellules.

La diminution conjointe de l'expression de multiples kinésines pourrait donc être une conséquence d'une chute de la concentration d'AMPc suite à une stimulation par l'extrait n°1 de l'activité phosphodiestérase.

La diminution d'expression et par hypothèse de l'activité de transport antérograde des mélanosomes via les kinésines, favorisant l'agrégation de ceux-ci et inhibant leur transfert, est donc à la base de notre hypothèse soutenant un effet dépigmentant de l'extrait n°1.

Une diminution de production de KGF/FGF-7 par les fibroblastes (Tableau 7) renforce cette hypothèse, ce dernier étant en effet un élément clé nécessaire à l'activation du processus de phagocytose des mélanosomes via le récepteur tyrosine kinase FGFR2b/KGFR lors du processus de transfert.

Ainsi, l'extrait n°1 pourrait dès lors être à la base d'une allégation dépigmentante et d'une stratégie originale permettant d'atténuer les taches pigmentaires apparaissant avec l'âge (lentigo solaire), caractérisées par une hyperpigmentation locale, et ce au travers d'un mécanisme original basé sur la régulation de la communication paracrine entre le compartiment dermique et épidermique visant à stimuler la mélanogénèse.

De plus, les deux extraits présentent des effets complémentaires par rapport à une possible allégation dépigmentante. En effet, l'extrait n°2 agit sur plusieurs facteurs mélanogéniques au niveau de l'épiderme (mélanocytes et kératinocytes), tandis que l'extrait n°1 agit de façon remarquable au niveau des fibroblastes du derme sur les 3 facteurs de croissance connus pour agir de façon paracrine sur les mélanocytes et les kératinocytes de l'épiderme et impliqués dans l'apparition des taches pigmentaires liées à l'âge.

A la fois cette complémentarité et l'action conjointe sur les mêmes cibles suggèrent un intérêt évident à mettre en place une stratégie visant à combiner les deux extraits, de manière à renforcer les effets attendus.

### • Elasticité et résistance mécanique de la peau :

Comme pour l'extrait n°2, nous observons dans les mêmes proportions l'induction conjointe des trois sous-unités de la laminine 5, à savoir la laminine α3, la laminine β3) ainsi que la laminine γ2 (Tableau 8).

Ainsi, les deux extraits pourraient dès lors être à la base d'une allégation anti-âge en permettant d'atténuer la perte d'élasticité et de la résistance mécanique de la peau.

### 4.3. Conclusion

L'étude transcriptomique décrite dans le présent exemple montre la modification significative de l'expression de gènes impliqués dans un stress oxydant dans des cultures cellulaires de fibroblastes humain en réponse à l'application de l'extrait n°2, et ainsi illustre le rôle protecteur des esters de DPG de DCQ contre ce stress oxydant.

Egalement, comme montré dans le présent exemple, les esters de DPG de DCQ et les esters de propane 1,3 diol de DCQ présents respectivement dans l'extrait n°2 et l'extrait n°1 ont modifié de façon significative l'expression de gènes impliqués dans la pigmentation cutanée et la jonction dermo-épidermique dans des cultures d'épidermes humains reconstitués mélanisés.

Ainsi, les deux extraits utilisés seuls ou en combinaison pour un effet complémentaire ou même synergique, pourraient dès lors être à la base d'une allégation anti-âge.

### RÉFÉRENCES BIBLIOGRAPHIQUES

Benilton S Carvalho And Rafael A Irizarry. Bioinformatics 2010 (Oxford University Press), 26:19, Pp 2363-7, Doi: 10.1093/Bioinformatics/Btq431.
Buckley & Klaassen, 2009
D. Gems, L. Partridge. Cell Metabolism, 2008, 7: 200-204
F. J. Kelly. Occup. Environ. Med., 2003, 60: 612-616
Gentleman R, Carey Vj, Bates Dm, Bolstad B, Dettling M, Dudoit S, Ellis B, Gautier L, Ge Y.Genome Biology 2004, 5, R80
Ihaka R and Gentleman T. Journal Of Computational And Graphical Statistics 1996, 5 (3), 299-314
Irizarry Ra, Hobbs B, Collin F, Beazer-Barclay Yd, Antonellis Kj, Scherf U, Speed Tp. Biostatistics. 2003b, 4 (2), 249-64
Irizarry Ra, Ooi SI, Wu Z, Boeke Jd. Stat Appl Genet Mol Biol. 2003a, 2, Epub. 2003 Mar 18
Kevin C. Kregel, Hannah J. Zhang. Am J Physiol Regul Integr Comp Physiol, 2007, 292:R18-R36
Lima et al., Mol. Nutr. Food Res., 54, 1-13, 2010; Calabrese et al., Clinics in Dermatology, 26, 358-363, 2008
Lu et al., 2009 ; Strange et al., 2001
Morse and Choi, 2005 ; Vile et al., 1994)
Murphy, 2013
Nguyen et al., 2009
Petri et al., 2012
Salmon M & Berger F. Expression Cosmétique 2014, 30, 91-94
S. I. Rattan. Ann. N. Y. Acad. Sci. 1998, 854: 54-60
Smyth Gk. Statistical Applications In Genetics And Molecular Biology 2004, 3 (3)
Suresh I. S. Rattan. The Journals of Gerontology Series A: Biological Sciences and Medical Sciences, 2004, 59:B705-B709
US 2004/0170581
WO 01/33942
Zsolt Radak, Hae Young Chung, Sataro Goto. Biogerontology, 2005, 6: 71-75

## Revendications

1. Composés selon la formule générale (I) 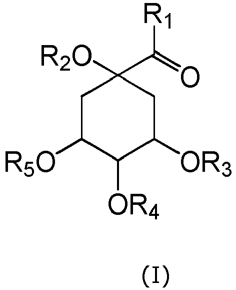 dans laquelle
R₁ représente un radical choisi dans le groupe constitué par les radicaux de formules (IIa) à (IIe), (III), (IVa) à (IVb) et (Va) à (Vd) suivantes : 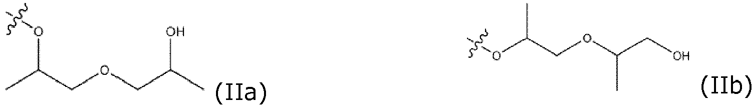 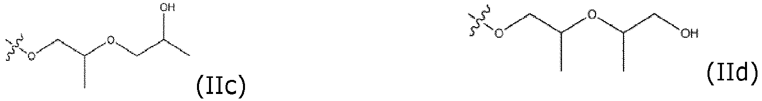 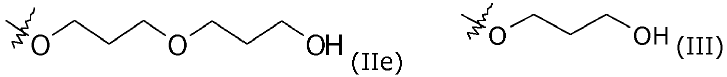 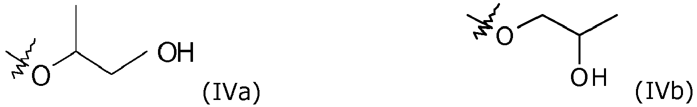 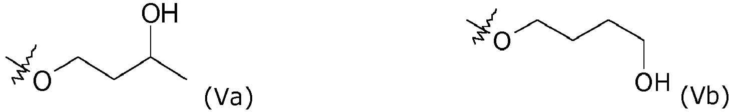 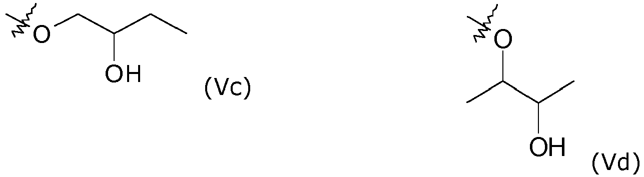 et dans laquelle deux quelconques des radicaux R₂, R₃, R₄ et Rs représentent un groupement caféoyle, les deux autres représentant un atome d'hydrogène.

2. Composés selon la revendication 1, **caractérisés en ce que** R₂ représente un atome d'hydrogène.

3. Composés selon la revendication 1, **caractérisés en ce que** R₂ et R4 représentent un atome d'hydrogène.

4. Composés selon l'une quelconque des revendications précédentes **caractérisés en ce qu'**ils sont sélectionnés parmi les molécules de formules respectives (Ia), (Ib), (Ic), (Id), (Ie), (If), (Ig), (Ih), (Ii), (Ij), (Ik) et (Il) suivantes :

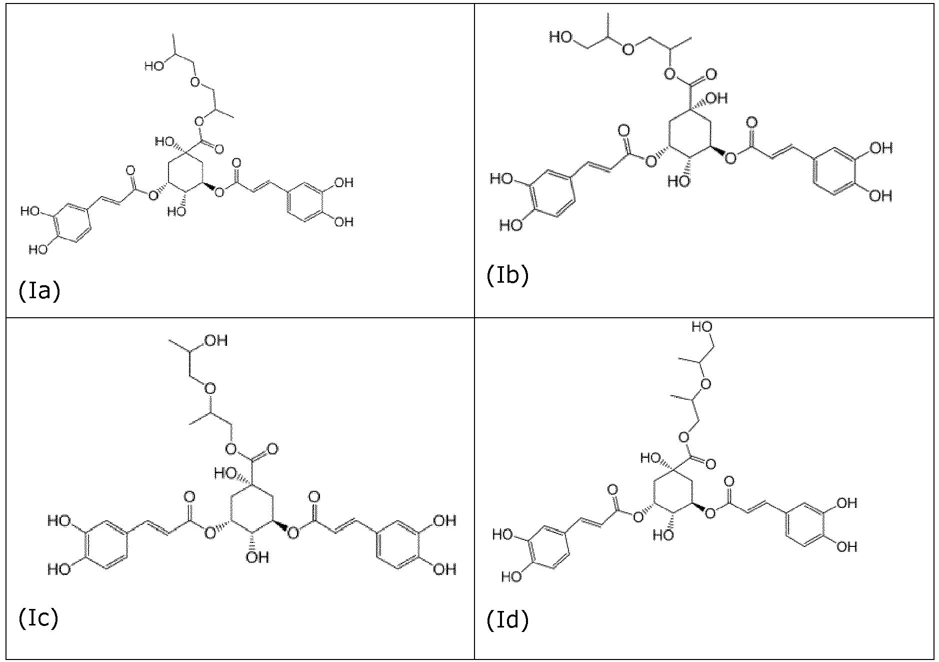

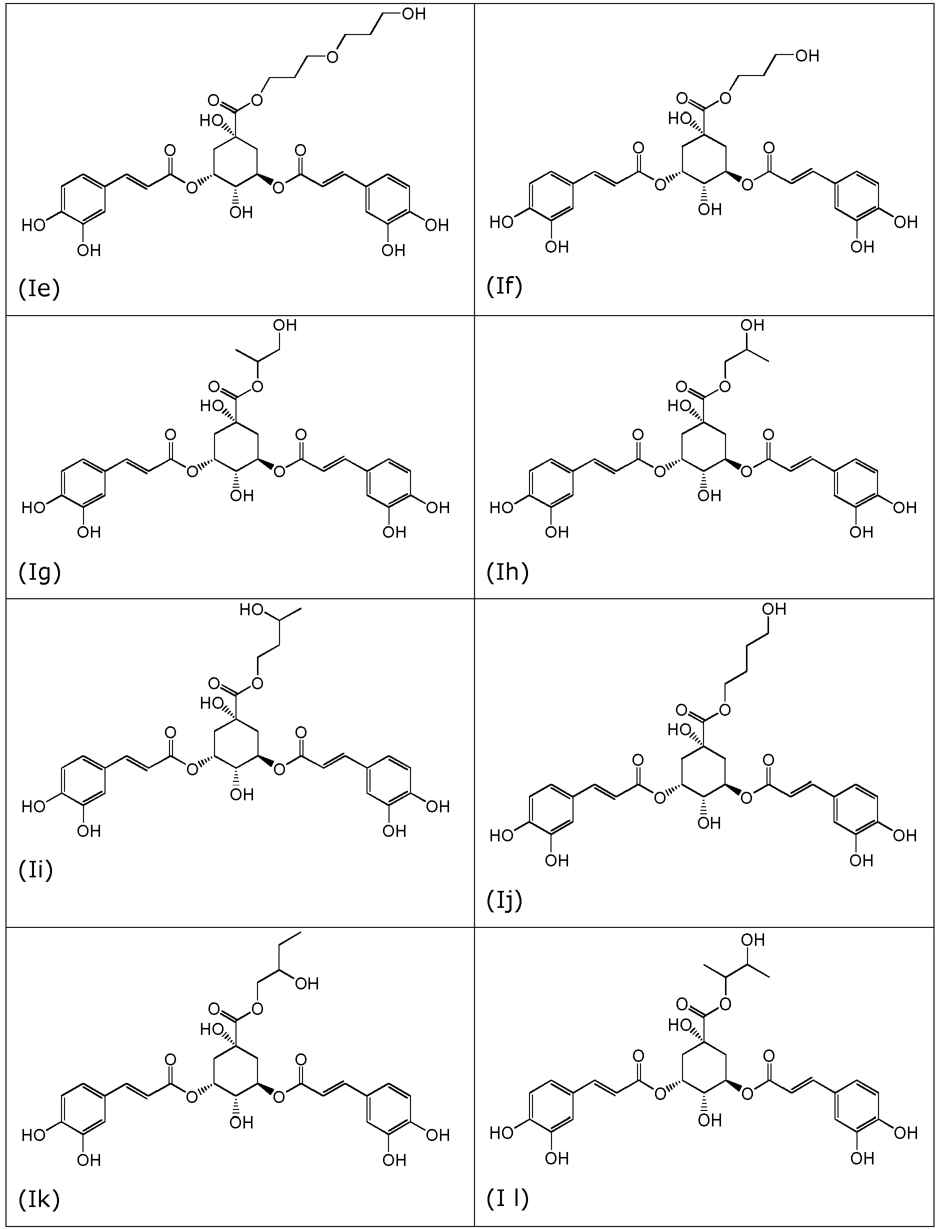

5. Extrait végétal comprenant au moins un composé selon l'une des revendications 1 à 4.

6. Extrait végétal selon la revendication 5, **caractérisé en ce qu'**il comprend en outre un acide dicaféoylquinique et/ou un acide chlorogénique.

7. Extrait végétal selon la revendication 6, **caractérisé en ce que** l'acide dicaféoylquinique est choisi parmi au moins l'un des composés suivant : l'acide 3,5-di-caféoylquinique, l'acide 3,4-di-caféoylquinique et l'acide 4,5-di-caféoylquinique.

8. Extrait végétal selon l'une quelconque des revendications 5 à 7, **caractérisé en ce que** l'extrait est un extrait *d'Ipomoea batatas,* notamment un extrait racinaire *d'Ipomoea batatas,* plus particulièrement choisi parmi : un extrait racinaire *d'Ipomoea batatas* obtenu par exsudation racinaire dans un solvant, un extrait racinaire *d'Ipomoea batatas* obtenu par macération racinaire dans un solvant et un mélange de ces deux extraits racinaires, ledit solvant étant constitué par un glycol pur ou par une solution, de préférence une solution aqueuse, de glycol.

9. Procédé de préparation d'un extrait végétal selon l'une quelconque des revendications 5 à 8, comprenant :
a. Une étape de culture de plantes synthétisant au moins un acide dicaféoylquinique, en particulier *d'Ipomoea batatas,* et
b. Une étape d'extraction solide/liquide des racines des plantes issues de l'étape a. dans un solvant choisi parmi : le dipropylène glycol, le propane 1,3 diol, le propane 1, 2 diol et le butylène glycol, ledit solvant étant utilisé pur ou sous la forme d'une solution aqueuse de glycol.

10. Procédé de préparation d'un extrait végétal selon la revendication 9, **caractérisé en ce que** l'étape b. est réalisée au moyen d'un procédé choisi parmi : une exsudation racinaire et une macération racinaire.

11. Procédé de préparation d'un extrait végétal selon l'une quelconque des revendications 9 ou 10, comprenant en outre les étapes suivantes :
• La section des racines
• Suivie éventuellement du séchage des racines.

12. Composition cosmétique comprenant en tant qu'agent actif au moins un composé selon l'une quelconque des revendications 1 à 4, un extrait selon l'une quelconque des revendications 5 à 8, ou un extrait obtenu par la mise en œuvre d'un procédé selon l'une quelconque des revendications 9 à 11, et avantageusement un excipient cosmétiquement acceptable.

13. Composé selon l'une quelconque des revendications 1 à 4, extrait selon l'une quelconque des revendications 5 à 8, extrait obtenu par la mise en œuvre d'un procédé selon l'une quelconque des revendications 9 à 11 ou composition cosmétique selon la revendication 12, pour prévenir le vieillissement cutané, pour atténuer les effets du vieillissement cutané, ou retarder l'apparition desdits effets, en particulier pour réduire et/ou retarder la formation de rougeurs, la formation des rides, la perte de fermeté de la peau, la perte de l'élasticité de la peau et la formation de taches de pigmentation de la peau, notamment les taches de pigmentation photo-induites et/ou les taches de sénescence.

14. Utilisation comme agent cosmétique d'au moins un composé selon l'une quelconque des revendications 1 à 4, d'un extrait selon l'une quelconque des revendications 5 à 8, d'un extrait obtenu par la mise en œuvre d'un procédé selon l'une quelconque des revendications 9 à 11 ou d'une composition cosmétique selon la revendication 12, pour prévenir le vieillissement cutané, pour atténuer les effets du vieillissement cutané, ou retarder l'apparition desdits effets, en particulier pour réduire et/ou retarder la formation de rougeurs, la formation des rides, la perte de fermeté de la peau, la perte de l'élasticité de la peau et la formation de taches de pigmentation de la peau, notamment les taches de pigmentation photo-induites et/ou les taches de sénescence.

15. Méthode de soin cosmétique de la peau visant à prévenir ou retarder l'apparition des effets du vieillissement de la peau, **caractérisée en ce qu'**elle comprend l'application sur au moins une partie de la peau du corps ou du visage d'une composition cosmétique selon la revendication 12.

## Patentansprüche

1. Verbindungen gemäß der allgemeinen Formel (I), 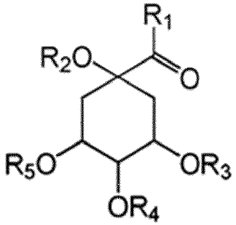 wobei
R₁ einen Rest darstellt, der ausgewählt ist aus der Gruppe bestehend aus den Resten der folgenden Formeln (IIa) bis (IIe), (III), (IVa) bis (IVb) und (Va) bis (Vd): 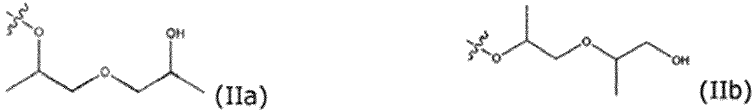 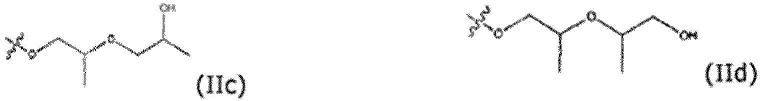 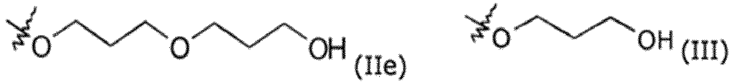 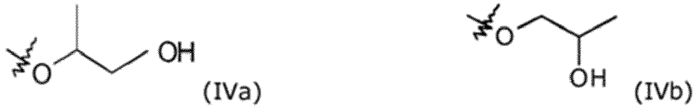 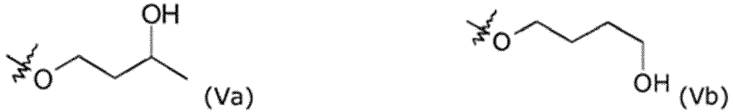 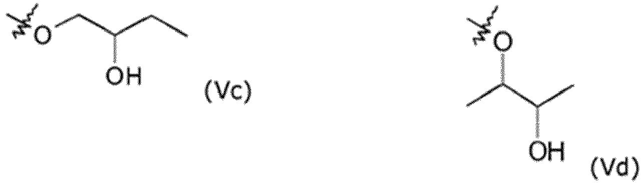 und wobei zwei beliebige der Reste R₂, R₃, R₄ und R₅ eine Caffeoylgruppe darstellen, wobei die beiden anderen ein Wasserstoffatom darstellen.

2. Verbindungen nach Anspruch 1, **dadurch gekennzeichnet, dass** R₂ ein Wasserstoffatom darstellt.

3. Verbindungen nach Anspruch 1, **dadurch gekennzeichnet, dass** R₂ und R₄ ein Wasserstoffatom darstellen.

4. Verbindungen nach einem der vorhergehenden Ansprüche, **dadurch gekennzeichnet, dass** sie aus den Molekülen mit den folgenden jeweiligen Formeln (Ia), (Ib), (Ic), (Id), (Ie), (If), (Ig), (Ih), (Ii), (Ij), (Ik) und (II) ausgewählt sind:

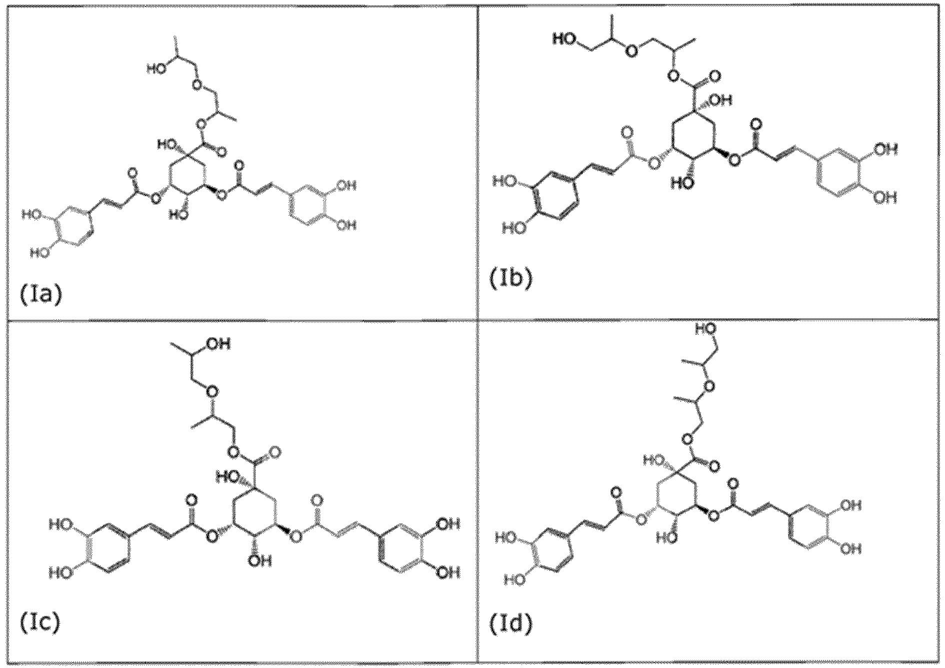

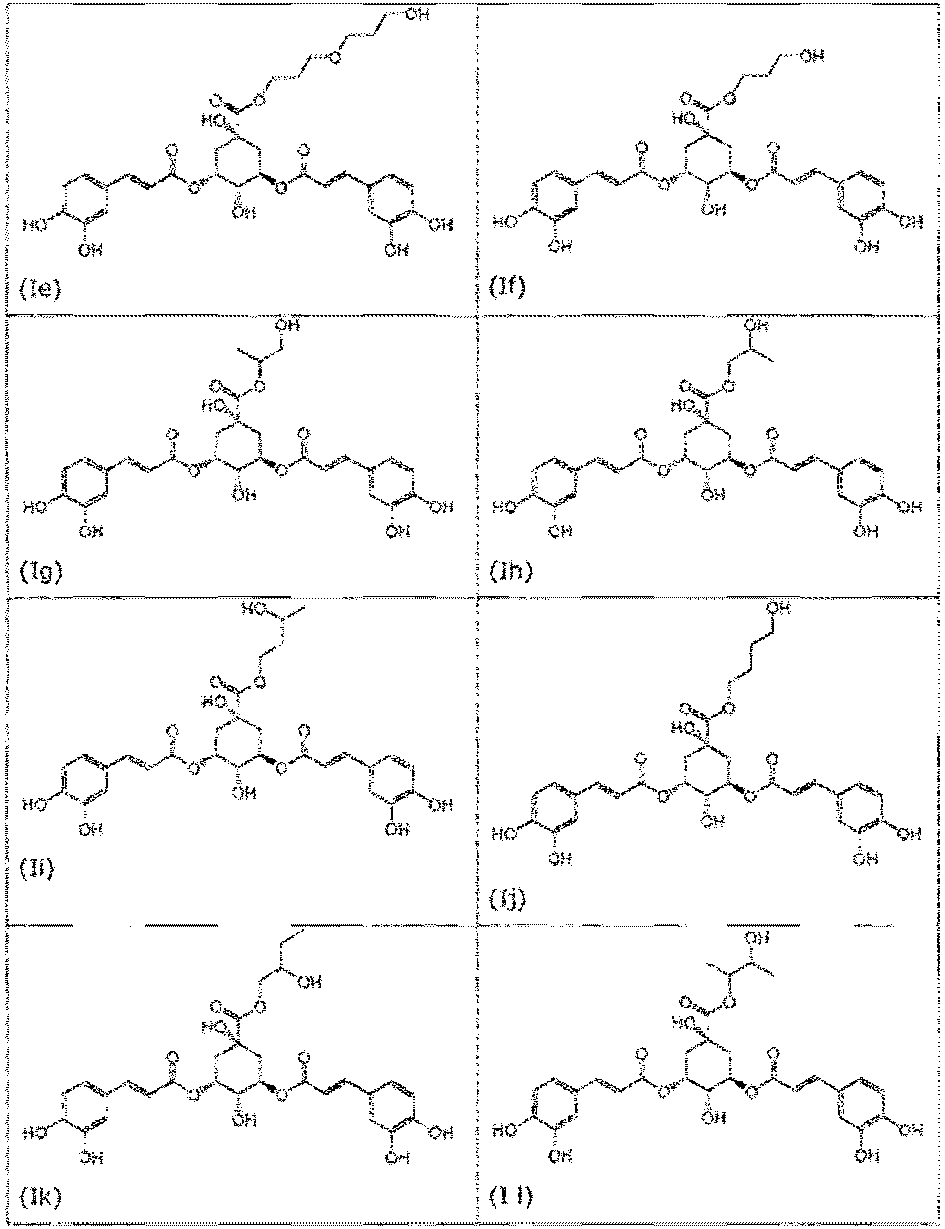

5. Pflanzlicher Extrakt, umfassend mindestens eine Verbindung nach einem der Ansprüche 1 bis 4.

6. Pflanzlicher Extrakt nach Anspruch 5, **dadurch gekennzeichnet, dass** er außerdem eine Dicaffeoylchinasäure und/oder eine Chlorogensäure umfasst.

7. Pflanzlicher Extrakt nach Anspruch 6, **dadurch gekennzeichnet, dass** die Dicaffeoylchinasäure aus mindestens einer der folgenden Verbindungen ausgewählt ist: 3,5-Dicaffeoylchinasäure, 3,4-Dicaffeoylchinasäure und 4,5-Dicaffeoylchinasäure.

8. Pflanzlicher Extrakt nach einem der Ansprüche 5 bis 7, **dadurch gekennzeichnet, dass** es sich beim Extrakt um einen Extrakt von *Ipomoea batatas,* insbesondere um einen Wurzelextrakt von *Ipomoea batatas* handelt, der noch mehr bevorzugt ausgewählt ist aus: einem Wurzelextrakt von *Ipomoea batatas,* der durch Wurzelexsudation in einem Lösungsmittel erhalten wird, einem Wurzelextrakt von *Ipomoea batatas,* der durch Wurzelmazeration in einem Lösungsmittel erhalten wird, und einem Gemisch dieser beiden Wurzelextrakte, wobei das Lösungsmittel aus einem reinen Glycol oder aus einer Lösung, vorzugsweise einer wässrigen Lösung, von Glycol besteht.

9. Verfahren zur Herstellung eines pflanzlichen Extrakts nach einem der Ansprüche 5 bis 8, umfassend:
a. einen Schritt der Kultivierung von mindestens eine Dicaffeoylchinasäure synthetisierenden Pflanzen, insbesondere von *Ipomoea batatas,* und
b. einen Schritt einer Fest-Flüssig-Extraktion von Wurzeln von Pflanzen aus Schritt a. in einem Lösungsmittel, das ausgewählt ist aus: Dipropylenglycol, 1,3-Propandiol, 1,2-Propandiol und Butylenglycol, wobei das Lösungsmittel rein oder in Form einer wässrigen Glycollösung verwendet wird.

10. Verfahren zur Herstellung eines pflanzlichen Extrakts nach Anspruch 9, **dadurch gekennzeichnet, dass** Schritt b. mittels eines Verfahrens erfolgt, das ausgewählt ist aus: einer Wurzelexsudation und einer Wurzelmazeration.

11. Verfahren zur Herstellung eines pflanzlichen Extrakts nach einem der Ansprüche 9 oder 10, umfassend außerdem die folgenden Schritte:
• das Schneiden der Wurzeln
• gegebenenfalls gefolgt vom Trocknen der Wurzeln.

12. Kosmetische Zusammensetzung, umfassend als Wirkstoff mindestens eine Verbindung nach einem der Ansprüche 1 bis 4, einen Extrakt nach einem der Ansprüche 5 bis 8 oder einen Extrakt, der durch die Ausführung eines Verfahrens nach einem der Ansprüche 9 bis 11 erhalten wird, und vorteilhafterweise einen kosmetisch annehmbaren Hilfsstoff.

13. Verbindung nach einem der Ansprüche 1 bis 4, Extrakt nach einem der Ansprüche 5 bis 8, Extrakt, der durch die Ausführung eines Verfahrens nach einem der Ansprüche 9 bis 11 erhalten wird, oder kosmetische Zusammensetzung nach Anspruch 12 zur Vorbeugung gegen Hautalterung, zur Linderung der Auswirkungen der Hautalterung oder zur Verzögerung des Auftretens der Auswirkungen, insbesondere zur Reduzierung und/oder Verzögerung der Bildung von Rötungen, der Bildung von Falten, des Verlusts der Festigkeit der Haut, des Verlusts der Elastizität der Haut und der Bildung von Pigmentflecken der Haut, insbesondere von photoinduzierten Pigmentflecken und/oder Altersflecken.

14. Verwendung als kosmetisches Mittel mindestens einer Verbindung nach einem der Ansprüche 1 bis 4, eines Extrakts nach einem der Ansprüche 5 bis 8, eines Extrakts, der durch die Ausführung eines Verfahrens nach einem der Ansprüche 9 bis 11 erhalten wird, oder einer kosmetischen Zusammensetzung nach Anspruch 12 zur Vorbeugung gegen Hautalterung, zur Linderung der Auswirkungen der Hautalterung oder zur Verzögerung des Auftretens der Auswirkungen, insbesondere zur Reduzierung und/oder Verzögerung der Bildung von Rötungen, der Bildung von Falten, des Verlusts der Festigkeit der Haut, des Verlusts der Elastizität der Haut und der Bildung von Pigmentflecken der Haut, insbesondere von photoinduzierten Pigmentflecken und/oder Altersflecken.

15. Verfahren zur kosmetischen Hautpflege zur Vorbeugung gegen oder die Verzögerung des Auftretens von Auswirkungen der Hautalterung, **dadurch gekennzeichnet, dass** es das Auftragen einer kosmetischen Zusammensetzung nach Anspruch 12 auf mindestens einen Teil der Haut des Körpers oder des Gesichts umfasst.

## Claims

1. Compounds having the general formula (I) 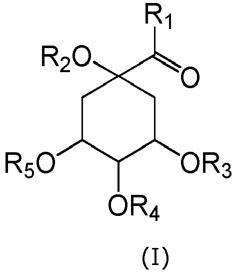 wherein
R₁ represents a radical chosen from the group consisting of the radicals of the following formulae (IIa) to (IIe), (III), (IVa) to (IVb) and (Va) to (Vd): 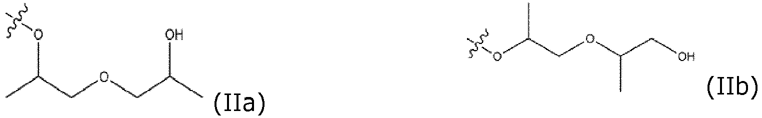 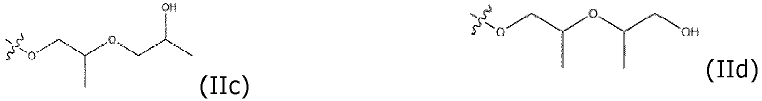 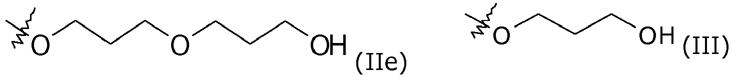 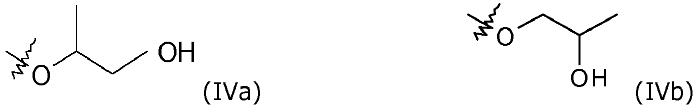 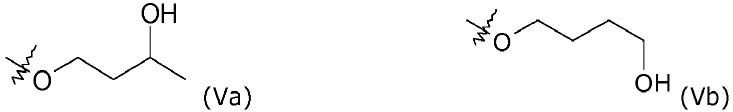 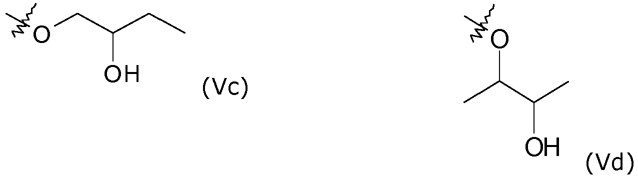 and wherein any two of the radicals R₂, R₃, R₄ and R₅ represent a caffeoyl group, the other two representing a hydrogen atom.

2. The compounds according to claim 1, **characterised in that** R₂ represents a hydrogen atom.

3. The compounds according to claim 1, **characterised in that** R₂ and R₄ represent a hydrogen atom.

4. The compounds according to any of the preceding claims, **characterised in that** they are selected from the molecules of the following respective formulae (Ia), (Ib), (Ic), (Id), (Ie), (If), (Ig), (Ih), (Ii), (Ij), (Ik) and (II):

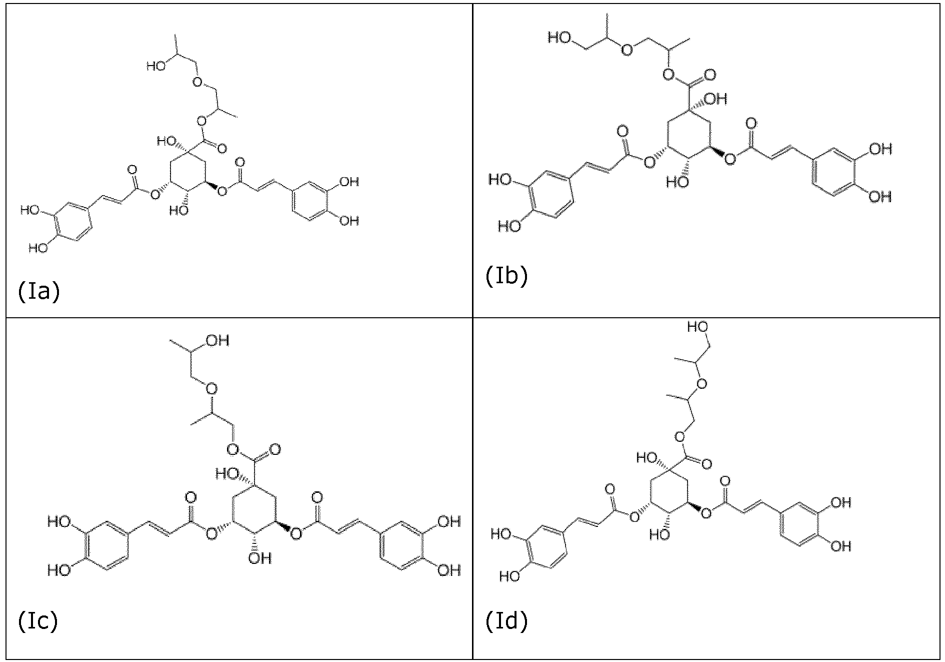

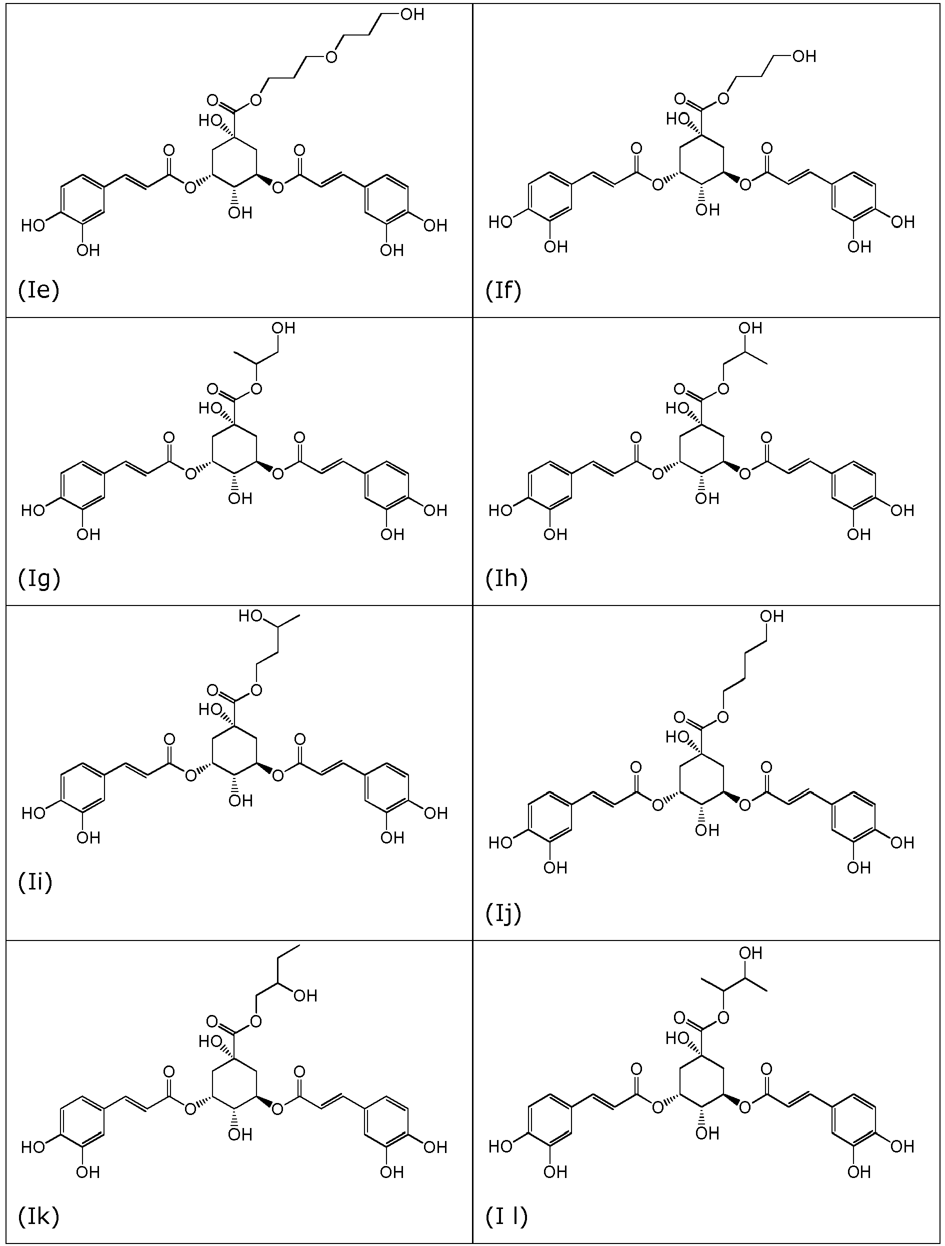

5. A plant extract comprising at least one compound according to any of claims 1 to 4.

6. The plant extract according to claim 5, **characterised in that** it further comprises a dicaffeoylquinic acid and/or a chlorogenic acid.

7. The plant extract according to claim 6, **characterised in that** the dicaffeoylquinic acid is chosen at least from one of the following compounds: 3,5-di-caffeoylquinic acid, 3,4-di-caffeoylquinic acid and 4,5-dicaffeoylquinic acid.

8. The plant extract according to any of claims 5 to 7, **characterised in that** the extract is an *Ipomoea batatas* extract, in particular an *Ipomoea batatas* root extract, more particularly chosen from: an *Ipomoea batatas* root extract obtained by root exudation in a solvent, an *Ipomoea batatas* root extract obtained by root maceration in a solvent and a mixture of both these root extracts, said solvent consisting of a pure glycol or of a solution, preferably an aqueous glycol solution.

9. A process for preparing a plant extract according to any of claims 5 to 8, comprising:
a. a step of culturing plants synthesising at least one dicaffeoylquinic acid, in particular of *Ipomoea batatas,* and
b. a step of solid/liquid extraction of plants roots from step a. in a solvent chosen from: dipropylene glycol, propane 1,3 diol, propane 1, 2 diol and butylene glycol, said solvent being used in pure form or as an aqueous glycol solution.

10. The process for preparing a plant extract according to claim 9, **characterised in that** step b. is made by means of a process chosen from: root exudation and root maceration.

11. The process for preparing a plant extract according to any of claims 9 and 10, further comprising the following steps of:
• severing the roots
• optionally followed by drying the roots.

12. A cosmetic composition comprising as an active agent at least one compound according to any of claims 1 to 4, an extract according to any of claims 5 to 8, or an extract obtained by implementing a process according to any of claims 9 to 11, and advantageously a cosmetically acceptable excipient.

13. The compound according to any of claims 1 to 4, the extract according to any of claims 5 to 8, the extract obtained by the implementation of a process according to any of claims 9 to 11 or the cosmetic composition according to claim 12, for preventing skin ageing, mitigating the effects of skin ageing, or delaying the appearance of said effects, in particular for reducing and/or delaying redness formation, wrinkle formation, loss of skin firmness, loss of skin elasticity and formation of skin pigmentation spots, in particular photo-induced pigmentation spots and/or senescence spots.

14. A use as a cosmetic agent of at least one compound according to any of claims 1 to 4, of an extract according to any of claims 5 to 8, of an extract obtained by the implementation of a process according to any of claims 9 to 11 or of a cosmetic composition according to claim 12, for preventing skin ageing, mitigating the effects of skin ageing, or delaying the appearance of said effects, in particular for reducing and/or delaying redness formation, wrinkle formation, loss of skin firmness, loss of skin elasticity and formation of skin pigmentation spots, in particular photo-induced pigmentation spots and/or senescence spots.

15. A skin cosmetic care method for preventing or delaying the appearance of skin ageing effects, **characterised in that** it comprises applying on at least a part of body or face skin a cosmetic composition according to claim 12.
